(19) Europäisches Patentamt
European Patent Office
Office européen des brevets

(11) Publication number: **0 082 648**
**A2**

(12) # EUROPEAN PATENT APPLICATION

(21) Application number: 82306610.5

(22) Date of filing: 10.12.82

(51) Int. Cl.[3]: C 07 D 501/18
C 07 D 498/04, C 07 D 471/04
A 61 K 31/545
//C07D233/68, C07D417/12,
C07D403/12, C07D401/12,
(C07D498/04, 265/00, 205/00),
(C07D471/04, 221/00, 205/00)

(30) Priority: 21.12.81 GB 8138390

(43) Date of publication of application:
29.06.83 Bulletin 83/26

(84) Designated Contracting States:
AT BE CH DE FR GB IT LI LU NL SE

(71) Applicant: IMPERIAL CHEMICAL INDUSTRIES PLC
Imperial Chemical House Millbank
London SW1P 3JF(GB)

(72) Inventor: Betts, Michael John
7 Beechway
Wilmslow Cheshire SK9 6LB(GB)

(72) Inventor: Ackerley, Norman
6 Overton Close
Congleton Cheshire CW12 1JZ(GB)

(72) Inventor: Stevenson, Robert
Boundary Cottage Leek Lane
Biddulph Moor Staffordshire ST8 7NE(GB)

(74) Representative: Brown, Ivor James Stewart et al,
Imperial Chemical Industries PLC Legal Department:
Patents Thames House North Millbank
London SW1P 4QG(GB)

(54) **Cephalosporin derivatives.**

(57) A cephalosporin derivative of the formula I:-

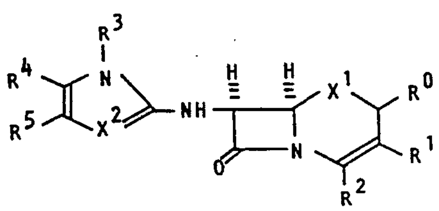

I

in which $R^0$ is hydrogen or methyl; $R^1$ is any one of the C-3 substituents from antibacterially-active cephalosporins known in the art; $R^2$ is any one of the C-4 substituents from antibacterially-active cephalosporins known in the art; $X^1$ is sulphur, oxygen or $CH_2$; $X^2$ is nitrogen or of the formula $\overset{\oplus}{N}$-$R^6$; $R^3$ and $R^6$, and $R^4$ and $R^5$, are a variety of substituents which are described in the specification; and the pharmaceutically-acceptable acid- or base-addition salts thereof. Pharmaceutical compositions and manufacturing processes are also described.

EP 0 082 648 A2

Croydon Printing Company Ltd

# CEPHALOSPORIN DERIVATIVES

This invention relates to cephalosporin derivatives which have antibacterial properties.

The vast majority of therapeuticaly useful antibiotics based on the penicillin and cephalosporin ring systems have an acylamino radical at the 6β and 7β positions respectively. A number of other substituents at these positions have been investigated but in the main the resulting compounds have at best possessed only weak antibacterial activity. One exception to this generalisation is the amidino substitutent. Penicillin derivatives carrying a substituted amidino radical in the 6β position (see for example UK patents 1,315,566 and 1,406,732) have been found to have useful antibacterial activity and two such compounds, mecillinam and pivmecillinam, are commercially available. However, the corresponding cephalosporin derivatives have been found to have a surprisingly low level of antibacterial activity. European Patent Publication No.18595 describes a series of cephalosporin derivatives carrying a 2- or 4-pyridinioamino radical in the 7-position.

European Patent Publications Nos.31708 and 55562 describe cephalosporin derivatives carrying a 7-(imidazolin-2-yl)amino or 7-(imidazol-2-yl)amino radical. The present invention represents an extension of this work to imidazole derivatives carrying a relatively complex carbon substituent.

According to the invention there is provided a cephalosporin derivative of the formula I:-

[Formula I - given hereafter]

in which $R^0$ is hydrogen or methyl;

$R^1$ is any one of the C-3 substituents from antibacterially-active cephalosporins known in the art;

$R^2$ is any one of the C-4 substituents from antibacterially-active cephalosporins known in the art;

$X^1$ is sulphur, oxygen or $CH_2$;

$X^2$ is nitrogen or of the formula $\overset{\oplus}{N}-R^6$;

$R^3$ and $R^6$, which may be the same or different, are hydrogen 1-6C alkyl, 1-6C alkanoyl, hydroxy, 1-6C alkoxy, amino, 1-6C alkanoylamino, 1-6C alkylamino, 1-6C aminoalkyl, 1-6C hydroxyalkyl, 2-6C carboxyalkyl, 2-6C alkenyl, 3-6C alkoxyalkyl, 3-8C alkoxycarbonylalkyl, furylmethyl, phenyl, 7-11C phenylalkyl,

in the latter two of which the phenyl ring is optionally substituted by halogen, methyl, methoxy, nitro, hydroxy, amino, carboxy or methoxycarbonyl,

3-6C alkynyl, 3-6C alkadienyl or 9-16C arylalkenyl;

one of $R^4$ and $R^5$ is hydrogen, 1-6C alkyl or 7-11C aralkyl and the other is of the formula II:-

[Formula II]

in which n is 1 to 6 and $-R^7$ is of the formula III:-

[Formula III]

in which $R^8$ is 2-6C hydroxyalkyl, 2-6C aminoalkyl, 3-10C alkylaminoalkyl, 4-15C dialkylaminoalkyl (or the N-oxide thereof), 2-6C cyanoalkyl, 3-6C (amino)-(carboxy)alkyl or heteroaryl,

or $-R^7$ is of the formula IV:-

[Formula IV]

in which $R^9$ is 2-6C aminoalkyl, p-nitrobenzyloxycarbonyl(2-6C aminoalkyl), 2-8C alkoxyalkyl, 3-10C alkoxycarbonylalkyl, 2-6C alkenyl, 1-6C haloalkyl, 2-6C carbamoylalkyl, 3-10C alkylcarbamoylalkyl, 4-15C dialkylcarbamoylalkyl, 2-6C cyanoalkyl, 6-10C aryl or heteroaryl,
or $-R^7$ is of the formula V:-

[Formula V]

in which $R^{10}$ is 6-10C aryl, furyl, thienyl, thiazolyl or thiadiazolyl, the latter two being optionally substituted by amino, and $R^{11}$ is hydrogen, 1-6C alkyl, 2-8C carboxyalkyl or 3-10C alkoxycarbonylalkyl,
or $-R^7$ is of the formula VI:-

[Formula VI]

in which $R^{12}$ is an acyl radical derived from an aminoacid or from two or three aminoacids linked via normal peptide bonds, the aminoacids (apart from glycine) being in either the D or L configuration and being selected from glycine, alanine, β-alanine, valine, leucine, isoleucine, serine, threonine, arginine, lysine, histidine, aspartic acid, glutamic acid, asparagine, glutamine, cysteine, methionine, phenylalanine, tyrosine, tryptophan and proline, the amino groups in such acyl radicals optionally carrying one or (where possible) more protecting groups selected from benzyloxycarbonyl, p-nitrobenzyloxycarbonyl, t-butoxycarbonyl and allyloxycarbonyl, the glutamic and aspartic acid residues optionally being in the form of the 1-6C alkyl esters and the arginine residue optionally being in the form of the nitro derivative,
or $-R^7$ is of the formula VII:-

[Formula VII]

in which -A- is of the formula VIII:-

[Formula VIII]

in which p is 1 or 2, or A is 3,4-dioxocyclobuten-1,2-diyl or of the formula C=Z in which Z is NCN, $NCONH_2$, $NNO_2$, $CHNO_2$ $C(CN)_2$, $NCOR^{14}$, $NCO_2R^{14}$, $SO_2R^{14}$ or $NR^{15}$ in which $R^{14}$ is 1-6C alkyl or 6-10C aryl and $R^{15}$ is 1-6C alkyl and $R^{13}$ is 1-6C alkoxy, 1-6C alkylthio, amino, 1-6C alkylamino or 2-10C dialkylamino;

wherein when $R^8$ or $R^9$ is heteroaryl it is a 5 or 6 membered heterocyclic aromatic ring containing 1,2,3 or 4 hetero atoms selected from oxygen, nitrogen and sulphur, and wherein when $R^8$ or $R^9$ is heteroaryl or $R^9$ is aryl the ring may be optionally substituted by one or two substituents selected from halogen, 1-6C alkyl, hydroxy, 1-6C alkoxy, mercapto, 1-6C alkylthio, carboxy, 2-6C alkoxycarbonyl, carbamoyl, 2-6C alkylcarbamoyl, 3-10C dialkylcarbamoyl, nitro, amino, 1-6C alkylamino, 2-8C dialkylamino, 1-6C alkanoylamino, 2-10C (alkanoyl)(alkyl)amino, cyano, sulphamoyl, 1-6C alkylsulphamoyl, 2-10C dialkylsulphamoyl, 1-6C haloalkyl, 1-6C alkanesulphinyl and 1-6C alkanesulphonyl;

provided that when $R^{10}$ is heteroaryl and m is 0 then n is 2 to 6:

and where the compound of the formula I contains a free basic or acidic group, the pharmaceutically-acceptable acid- and base-addition salts thereof.

It is to be understood that in the above formula I and throughout this specification, the illustrated stereochemistry of the ceph-3-em nucleus of the formula IX:-

[Formula IX]

is the absolute configuration. It is also to be understood that although the double bonds in the ring attached to the 7-amino group have been inserted in particular positions, other tautomeric forms are, in certain instances, possible. Note, however, that the delta-3 double bond is fixed in position. When $X^1$ is sulphinyl the oxygen atom may be in the $\alpha$ or $\beta$ configuration, or a mixture of both. When the compound of the formula I contains both an acidic and a basic centre, the compound may exist in the form of a zwitterion.

A particular value for $R^1$ is hydrogen, halogen (e.g. fluorine, chlorine or bromine), hydroxy or amino or a saturated or unsaturated, substituted or unsubstituted 1-20C organic group. Illustrative values for $R^1$ when it is a 1-20C organic group are as follows:-

(a) 1-6C alkyl, benzyl optionally substituted by fluorine or methoxy, 1-6C haloalkyl, formyl, carboxy, 1-6C alkoxy, 1-6C alkylthio, 1-6C alkylamino, phenylamino, benzylamino, 3-6C cycloalkylamino, cyano, 2-6C alkoxycarbonyl, 2-6C alkanoyl, 3-10C alkoxycarbonylalkyl, 2-6C alkoxycarbonylamino, 2-6C alkylthiocarbonylamino, piperidino, pyrrolidino, morpholino, 2-6C alkanoylamino, ureido, 2-6C alkylureido, 3-8C dialkylureido, 1-6C alkanesulphinyl, 1-6C alkanesulphonyl, heterocyclyl and heterocyclylthio in which the heterocycle is 1,3,4-thiadiazol-2-yl or 1,3,4-oxadiazol-2-yl, each optionally substituted in the 5-position, 1H-tetrazol-5-yl optionally substituted in the 1-position, or 1H-1,2,3-triazol-4-yl optionally substituted in the 1 or 5 position, the optional substituents in each of these heterocycles being 1-6C alkyl, 1-6C sulphoalkyl, 2-6C carboxyalkyl,

1-6C haloalkyl or 3-6C alkylthioalkyl, or pyridazin-3-yl, oxazol-3-yl or thiazol-3-yl each optionally substituted by 1 or 2 radicals selected from 1-6C alkyl, 1-6C haloalkyl and 2-6C alkoxycarbonyl;

(b) radicals of the formula X:-

[Formula X]

in which $R^{16}$ and $R^{17}$, which may be the same or different, are hydrogen, 1-6C alkyl, 5-7C cycloaliphatic, 6-12C aryl, 7-10C arylalkyl (e.g. benzyl, 2-phenethyl), formyl, cyano, carboxy, 2-6C alkoxycarbonyl, sulpho, 1-6C alkanesulphinyl, 1-6C alkanesulphonyl, 1-6C alkoxy, 1-6C alkylthio, carbamoyl, nitro, 1-6C hydroxyalkyl, methylcarbamoyloxymethyl, benzylcarbamoyloxymethyl, 2-6C alkoxymethyl, 2-6C alkylthiomethyl, 2-haloethoxymethyl, cyclopentyloxymethyl, benzyloxymethyl or 3-8C alkanoyloxymethyl or radicals of the formula $CH_2SHet^1$ in which $Het^1$ is 1,3,4-thiadiazol-2-yl or 1,3,4-oxadiazol-2-yl both optionally substituted in the 5-position by methyl, 1H-triazol-5-yl optionally substituted in the 1-position by methyl or 1H-1,2,3-triazol-4-yl;

(c) radicals of the formula XI:-

[Formula XI]

in which $R^{18}$ is cyano, carboxy or 2-6C alkoxycarbonyl;

(d) radicals of the formula XII:-

[Formula XII]

in which $R^{19}$ and $R^{20}$, which may be the same or different, are hydrogen or 1-6C alkyl and e is 1 to 4;

(e) radicals of the formula $CH_2Y$ in which Y is an atom or group which is the residue of a nucleophile or a derivative of a residue of a nucleophile, such a nucleophile or a derivative thereof being:-

A. 3-15C trialkylamines;

B. heterocyclic amines having more than one heteroatom, at least one heteroatom being nitrogen;

C. pyridines which ar optionally substituted by 1 to 3 substituents selected from halogen, 1-6C alkyl, 6-10C aryl, 7-11C arylalkyl, 2-10C alkoxyalkyl, 3-10C alkanoyloxymethyl, formyl, carbamoyl, 2-6C alkanoyloxy, 2-6C alkoxycarbonyl, 1-6C alkoxy, 6-10C aryloxy, 7-11C aralkoxy, 1-6C alkylthio, 6-10C arylthio, 7-11C aralkylthio, cyano, hydroxy, 2-6C alkylcarbamoyl, 3-10C dialkylcarbamoyl, 2-6C (hydroxyalkyl)carbamoyl and 2-6C carbamoylalkyl;

D. azide;

E. amino, 1-6C alkanoylamino and 7-11C aroylamino;

F. cyanide, pyrrole and substituted pyrroles;

G. nucleophiles giving rise to $R^1$ of the formula XIII:-

[Formula XIII]

in which $R^{21}$ and $R^{22}$, which may be the same or different, are selected from hydrogen, cyano, 1-6C alkyl, 2-6C alkoxycarbonyl, 8-20C mono- or di-arylalkoxycarbonyl, 2-6C alkanoyl, 7-11C aralkyl, cyclopentyl and cyclohexyl, and phenyl optionally substituted by 1 or 2 radicals selected from halogen, 1-6C alkyl, 1-6C alkoxy, 1-6C alkylamino, nitro and amino, and $R^{23}$ is selected from hydrogen, 1-6C alkyl, 7-11C aralkyl, cyclopentyl and cyclohexyl, and phenyl

optionally substituted by 1 or 2 radicals selected from halogen, 1-6C alkyl, 1-6C alkoxy and 1-6C alkylamino;

H. thiourea optionally substituted by 1-6C alkyl, 6-10C aryl, 5-7C alicyclic or heterocyclyl, dithiocarbamates, thioamides substituted by 1-6C alkyl or 6-10C aryl or thiosemicarbazides, thiosulphates, arylthioacids or heterocyclicthioacids of up to 10 carbons and dithioacids of the formula XIV:-

[Formula XIV]

in which $R^{24}$ and $R^{25}$, which may be the same or different, are hydrogen, 1-6C alkyl, 2-6C hydroxyalkyl, 3-8C alkylaminoalkyl, 4-10C dialkylaminoalkyl or phenyl, or $R^{24}$ and $R^{25}$ are joined to form a pyrrolidine, piperidine or morpholine ring or a piperazine ring which is optionally substituted on nitrogen by one or two (in quaternised form) radicals selected from 1-6C alkyl and 3-6C alkenyl;

I. compounds of the formula $R^{26}S(O)_dH$ in which d is 0, 1 or 2 and $R^{26}$ is 1-6C alkyl, 5-7C alicyclic, 6-10C aryl optionally substituted by carboxy, or 7-11C arylalkyl or a 5- or 6-membered heterocyclic ring (partially or fully unsaturated) containing 1 to 4 nitrogens which ring may further include (where possible) oxygen and/or sulphur, in which the nitrogen may be in the oxide form, which heterocylic ring may be fused with another heterocyclic ring within the same definition or may be fused with a benzene ring, the above aryl, arylalkyl, heterocyclic or fused benzene ring being optionally substituted (where possible) by 1 or 2 substituents selected from halogen, 1-6C alkyl, 1-6C haloalkyl, 6-10C aryl, 2-6C alkenyl, 1-6C alkoxy, oxo, hydroxy, mercapto, amino, carboxy, cyano, isothiocyanate, carbamoyl, sulphamoyl, 2-6C alkoxycarbonyl, 3-6C alkenyloxycarbonyl, 8-12C

aralkylcarbonyl, 7-11C aryloxycarbonyl, 2-6C hydroxyalkyl, 3-6C dihydroxyalkyl, sulphoamino and 1-6C alkanesulphonylamino and radicals of the formula B-$R^{27}$ in which B is a 2-8C straight or branched chain which may be interrupted by sulphur, oxygen, NH or 1-6C N-alkyl and $R^{27}$ is selected from hydroxy, mercapto, cyano, 1-6C alkylamino, 2-6C dialkylamino, 2-6C alkanoylamino, carboxy, sulpho, carbamoyl, sulphamoyl, amidino, guanidino, 2-6C alkoxycarbonyl, 2-6C alkylcarbamoyl, 2-6C dialkylcarbamoyl, 1-6C alkylsulphamoyl, 2-6C dialkylsulphamoyl, sulphoamino, ureido, 1-6C alkoxy, 1-6C alkylthio, 1-6C alkanesulphonyl, 2-6C alkanoyl and 2-6C alkanoyloxy and radicals of the formula -S-$R^{28}$ in which $R^{28}$ is 1-6C alkyl or a group of the formula B-$R^{27}$ in which B and $R^{27}$ have the meanings given above and radials of the formula $NR^{29}R^{30}$ in which $R^{29}$ and $R^{30}$, which may be the same or different, are selected from 1-6C alkyl, groups of the formula B-$R^{27}$ in which B and $R^{27}$ have the definitions given above, 1-6C alkoxycarbonyl, 2-6C alkanoyl, carbamoyl, 2-6C alkylcarbamoyl and 3-10C dialkylcarbamoyl;

J. radicals of the formula $R^{31}$-OH in which $R^{31}$ is hydrogen, 1-6C alkyl, 3-6C alkenyl, 3-6C alkynyl, 5-7C cycloalkyl, 6-12C cycloalkylalkyl, 6-10C aryl, 7-11C arylalkyl or furfuryl, any of which may be substituted by 1 or 2 radicals selected from halogen, 1-6C alkyl, nitro, hydroxy, carboxy, 2-6C alkanoyloxy, 2-6C alkoxycarbonyl, 2-6C alkanoyl, 1-6C alkanesulphonyl, 1-6C alkoxysulphonyl, amino, 1-6C alkylamino and 2-6C alkanoylamino, or $R^{31}$ is carbamoyl;

K. radicals of the formula $R^{32}$-Q-COOH in which Q is a direct bond, oxygen, sulphur or NH and $R^{32}$ is:-

(i) hydrogen or 1-6C alkyl which may be interrupted by oxygen, sulphur or NH or substituted by a cyano, carboxy, methoxycarbonyl, ethoxycarbonyl, hydroxy, carboxycarbonyl, halogen or amino;

(ii) 2-6C alkenyl which may be interrupted by oxygen, sulphur or NH;

(iii) phenyl, hydroxyphenyl, chlorophenyl, fluorophenyl, tolyl, nitrophenyl, aminophenyl, methoxyphenyl, methylthiophenyl, thienyl, pyridyl, cyclohexyl, cyclopentyl, sydnonyl, naphthyl or ethoxynaphthyl;

or (iv) $R^{33}$-$(CH_2)_g$ where $R^{33}$ has the value for $R^{32}$ listed in (i) above and g is 1 to 4;

and (f) radicals of the formula XV:-

[Formula XV]

in which $R^{34}$ is

1) 1-6C alkyl (e.g. methyl), L-2-amino-2-carboxyethyl or phenyl;

2) pyridyl or the N-oxide thereof;

3) pyridazin-3-yl substituted in the 6-position by 1-6C alkyl (e.g. methyl), methoxy, amino or 1-6C acylamino (e.g. acetylamino), or the N-oxide thereof, or pyrimidin-2-yl or tetrazolo[4,5-b]pyridazin-6-yl;

4) 5,6-dioxo-1,4,5,6-tetrahydro-1,2,4-triazin-3-yl substituted in the 4-position; 1,3,4-triazol-5-yl or 2-alkoxycarbonyl-1,3,4-triazol-5-yl in which the alkoxycarbonyl is 2-6C (e.g. methoxycarbonyl), each substituted in the 1-position:-

(a) by 1-6C alkyl (e.g. methyl), optionally substituted by 1-6C alkoxy (e.g. methoxy), 1-6C alkylthio (e.g. methylthio), phenyl, formyl, carbamoyl, 2-6C alkylcarbamoyl (e.g. methylcarbamoyl), 3-10C dialkylcarbamoyl

(e.g. dimethylcarbamoyl), 1-6C alkanoyl (e.g. acetyl), 2-6C alkoxycarbonyl (e.g. methoxycarbonyl) or thiazolidin-2-yl;
(b) by allyl, 2,3-dihydroxypropyl, 1,3-dihydroxyprop-2-yl, 2-formyl-2-hydroxyethyl, 3-formyloxy-2-hydroxypropyl, 2,3-bisformyloxypropyl or 1,3-bisformyloxyprop-2-yl;
(c) by 2-4C alkyl (e.g. ethyl) substituted by hydroxy, carbamoyloxy, 1-6C alkanoyl (e.g. acetyl) (which can itself be optionally substituted by amino, 1-6C alkylamino [e.g. methylamino] or 2-10C dialkylamino [e.g. dimethylamino]), 1-6C alkanesulphinyl (e.g. methanesulphinyl), 1-6C alkanesulphonyl (e.g. methanesulphonyl), amino, 1-6C alkylamino (e.g. methylamino), 2-10C dialkylamino (e.g. dimethylamino), sulphoamino, 1-6C alkanesulphonylamino, (e.g. methanesulphonylamino), sulphamoylamino, 1-6C alkanoylamino (e.g. acetylamino) (which can itself be optionally substituted by hydroxy, amino, 1-6C alkylamino [e.g. methylamino] or 2-10C dialkylamino [e.g. dimethylamino]), 2-6C alkoxycarbonylamino (e.g. methoxycarbonylamino), ureido, 2-6C alkylureido (e.g. methylureido), or 3-10C dialkylureido (e.g. dimethylureido);
(d) by a radical of the formula XVI, XVII or XVIII:-

[Formula XVI]

[Formula XVII]

[Formula XVIII]

in which alk is 1-4C alkylene (e.g. methylene), $Y^1$ and $Y^2$ are the same and are oxygen or sulphur and $R^{35}$ and $R^{36}$ are the same and are 1-6C alkyl (e.g. methyl), or $Y^1$ and $Y^2$ are the same or different and are oxygen or sulphur and $R^{35}$ and $R^{36}$ are joined to form 2-3C alkylene (e.g. ethylene), and $R^{37}$ is hydrogen or 1-3C alkyl (e.g. methyl);

(e) by 1-6C alkyl (e.g. methyl) substituted by 1-6C alkoxyimino (e.g. methoxyimino) or hydroxyimino;

5) 1,4-dialkyl-5,6-dioxo-1,4,5,6-tetrahydro-1,2,4-triazin-3-yl, 1-alkyl-5,6-dioxo-1,4,5,6-tetrahydro-1,2,4-triazin-3-yl or 2-alkyl-5,6-dioxo-1,2,5,6-tetrahydro-1,2,4-triazin-3-yl in each of which the alkyl is 1-6C (e.g. methyl);

6) 1,3,4-triazol-5-yl, 1,2,3-triazol-5-yl or 1-alkyl-1,2,4-triazol-5-yl in which the alkyl is 1-6C (e.g. methyl) which is optionally substituted in the 3-position by 2-6C alkoxycarbonyl (e.g. methoxycarbonyl);

7)a. 1,3,4-thiadiazol-5-yl optionally substituted by 1-6C alkyl (e.g. methyl), trifluoromethyl, 1-6C alkoxy (e.g. methoxy), 1-6C alkylthio (e.g. methylthio), 2-4C hydroxyalkylthio (e.g. 2-hydroxyethylthio), 1-6C alkanesulphonyl (e.g. methanesulphonyl), hydroxy, 1-6C hydroxyalkyl (e.g. hydroxymethyl), carboxy, 2-6C carboxyalkyl (e.g. carboxymethyl), amino, 1-6C alkylamino (e.g. methylamino), 2-10C dialkylamino (e.g. dimethylamino), 1-6C aminoalkyl (e.g. aminomethyl), 2-8C alkylaminoalkyl (e.g. methylaminomethyl), 3-12C dialkylaminoalkyl (e.g. dimethylaminomethyl), 1-6C alkanoylamino (e.g. acetylamino) or 2-8C alkanoylaminoalkyl (e.g. acetylaminomethyl), or

b. 1,2,4-thiadiazol-5-yl substituted by 1-6C alky (e.g. methyl) or 1-6C alkoxy (e.g. methoxy);

8)a. 1,3,4-oxadiazol-5-yl optionally substituted by 1-6C alkyl (e.g. methyl), trifluoromethyl, phenyl, 1-6C aminoalkyl (e.g. aminomethyl), 2-8C alkylaminoalkyl (e.g. methylaminomethyl), 3-10C dialkylaminoalkyl (e.g. dimethylaminomethyl) or 2-8C alkanoylaminoalkyl (e.g. acetylaminomethyl) or

b. oxazol-2-yl optionally substituted in the 4-position by 1-6C alkyl (e.g. methyl);

9) tetrazol-5-yl optionally substituted in the 1-position by:-

(a) 1-6C alkyl (e.g. methyl) itself optionally substituted by 1-6C alkoxy (e.g. methoxy), sulpho, carboxy, formyl or sulphamoyl;

(b) 2-4C alkyl (e.g. ethyl) substituted by hydroxy, amino, 1-6C alkylamino (e.g. methylamino), 2-8C dialkylamino (e.g. dimethylamino), 1-6C alkanoylamino (e.g. acetylamino), 2-6C carboxyalkylamino (e.g. carboxymethylamino), sulphamoylamino, sulphoamino, ureido, 2-6C alkylureido (e.g. methylureido) or 3-8C dialkylureido (e.g. dimethylureido);

(c) 1-5C alkyl (e.g. methyl) substituted by hydroxyimino or 1-6C alkoxyimino (e.g. methoxyimino);

(d) phenyl, 2,3-dihydroxypropyl, 1,3-dihydroxyprop-2-yl, 2-formyl-2-hydroxyethyl, 3-formyloxy-2-hydroxypropyl, 2,3-bisformyl-oxypropyl or 1,3-bisformyloxy-2-propyl;

or

(e) a radical of the formula XVI above in which $R^{37}$ is hydrogen, or a radical of the formula XVII above, in both of which $Y^1$, $Y^2$, $R^{35}$ and $R^{36}$ are as given above.

A particular value for $R^2$ is carboxy, tetrazol-5-yl or a radical of the formula XIX, XX, XXI, XXII, XXIII, XXIV, XXV, XXVI, XXVII, XXVIII or XXIX:-

[Formula XIX]

[Formula XX]

[Formula XXI]

[Formula XXII]

[Formula XXIII]

[Formula XXIV]

[Formula XXV]

[Formula XXVI]

[Formula XXVII]

[Formula XXVIII]

[Formula XXIX]

in which $R^{38}$ is hydrogen or 1-6C alkyl, $R^{39}$ is 1-6C alkyl, $R^{40}$ is hydrogen, 1-6C alkyl, 7-11C arylalkyl or 2-6C alkoxycarbonyl, t is 0 or 1, $R^{41}$ is 1-6C alkyl, 6-10C aryl or 7-11C aralkyl, $R^{42}$ is hydrogen or one, two or three radicals selected from halogen, nitro, cyano, 1-6C alkyl, 1-6C alkoxy, 1-6C alkylthio, 1-6C alkylsulphinyl, 1-6C alkanesulphonyl, 2-6C alkoxycarbonyl, 2-6C alkoxythio-carbonyl, 2-6C alkanoylamino, 6-10C aryl, 6-10C aryloxy,

6-10C arylthio, 6-10C arylsulphinyl, 6-10C arylsulphonyl, 7-11C aryloxycarbonyl, 7-11C arylthiocarbonyl and 7-11C aryloxythiocarbonyl, $R^{43}$ is hydrogen or one of the values for $R^{41}$ given above and $R^{44}$ is hydrogen or one, two or three radicals selected from halogen, 1-6C alkyl and 1-6C alkoxy.

Particular values for $R^3$ and $R^6$, which may be the same or different, are hydrogen, methyl, acetyl, hydroxy, methoxy, amino, acetylamino, methylamino, 2-aminoethyl, 2-hydroxyethyl, carboxymethyl, 2-carboxyethyl, 3-carboxypropyl, allyl, methoxymethyl, methoxycarbonylmethyl, t-butoxycarbonylmethyl, 3-(ethoxycarbonyl)propyl, 3-(t-butoxycarbonyl)propyl, furylmethyl, phenyl, benzyl,

in the latter two of which the phenyl ring is optionally substituted by fluorine, chlorine, bromine, methyl, methoxy, nitro, hyroxy, amino, carboxy or methoxycarbonyl,

propargyl, allenyl or 3-phenylallyl.

A particular value for one of $R^4$ and $R^5$ is hydrogen, methyl or benzyl.

A particular value for $R^8$ is 2-hydroxyethyl, 2-aminoethyl, 2-methylaminoethyl, 2-dimethylaminoethyl (and the N-oxide thereof), 2-cyanoethyl or 2-amino-2-carboxyethyl, or furyl, thienyl, pyrrolyl, oxazolyl, thiazolyl, imidazolyl, pyrazolyl, isoxazolyl, isothiazolyl, thiadiazolyl, oxadiazolyl, triazolyl, tetrazolyl, pyridyl, pyrimidyl, pyrazinyl or pyridazinyl, each optionally substituted by one or two substituents selected from fluorine, chlorine, bromine, methyl, hydroxy, methoxy, mercapto, methylthio, carboxy, methoxycarbonyl, carbamoyl, methylcarbamoyl, dimethylcarbamoyl, nitro, amino, methylamino, dimethylamino, acetylamino, (acetyl)(methyl)amino, cyano, sulphamoyl, methylsulphamoyl, dimethylsulphamoyl, trifluoromethyl, methanesulphinyl and methanesulphonyl.

A particular value for $R^9$ is 3-aminopropyl, 3-(p-nitrobenzyloxycarbonylamino)propyl, 2-methoxyethyl, ethoxycarbonylmethyl, allyl, trifluoromethyl, carbamoylmethyl, methylcarbamoylmethyl, dimethylcarbamoylmethyl or 2-cyanoethyl, or phenyl, furyl, thienyl, pyrrolyl, oxazolyl, thiazolyl, imidazolyl, pyrazolyl, isoxazolyl, isothiazolyl, thiadiazolyl, oxadiazolyl, triazolyl, tetrazolyl, pyridyl, pyrimidyl, pyrazinyl or pyridazinyl each optionally substituted by one or two substituents selected from fluorine, chlorine, bromine, methyl, hydroxy, methoxy, mercapto, methylthio, carboxy, methoxycarbonyl, carbamoyl, methylcarbamoyl, dimethylcarbamoyl, nitro, amino, methylamino, dimethylamino, acetylamino, (acetyl)(methyl)amino, cyano, sulphamoyl, methylsulphamoyl, dimethylsulphamoyl, trifluoromethyl, methanesulphinyl and methanesulphonyl.

A particular value $R^{10}$ is phenyl, furyl, thienyl, thiazolyl or thiadiazolyl, the latter two being optionally substituted by amino.

A particular value for $R^{11}$ is hydrogen, methyl, 1-carboxy-1-methylethyl or 1-ethoxycarbonyl-1-methylethyl.

A particular value for $R^{12}$ is Gly, D-Ala, L-Ala, D-β-Ala, L-β-Ala, D-Val, L-Val, D-Leu, L-Leu, D-Ser, L-Ser, D-Thr, L-Thr, D-Arg, L-Arg, D-Lys, L-Lys, D-His, L-His, D-Asp, L-Asp, D-Glu, L-Glu, D-Asn, L-Asn, D-Gln, L-Gln, D-Lys, L-Lys, D-Met, L-Met, D-Phe, L-Phe, D-Tyr, L-Tyr, D-Trp, L-Trp, D-Pro, L-Pro or D-Ala-D-Ala, all the above residues optionally carrying a benzyloxycarbonyl, p-nitrobenzyloxycarbonyl, t-butoxycarbonyl or allyloxycarbonyl radical, the Glu and Asp residues optionally being in the form of the methyl or ethyl esters and the Arg residues optionally being in the form of the nitro derivative.

A particular value for $R^{14}$ is methyl or phenyl.

A particular value for $R^{15}$ is methyl.

A particular value for $R^{13}$ is methoxy, methylthio, amino, methylamino or dimethylamino.

The following are nine preferred features of the cephalosporin derivative of the formula I. When any one of these features is taken, either singly or in combination, with the other general or particular features of the cephalosporin derivative of the formula I listed above there are obtained preferred sub-groups within the above definition.

1. $X^1$ is sulphur.

2. $R^0$ is hydrogen.

3. $R^1$ is hydrogen, chlorine, methyl, acetoxymethyl, 1-methyl-1H-tetrazol-5-ylthiomethyl, 1-carboxymethyl-1H-tetrazol-5-ylthiomethyl, 1-(2-dimethylamino)-ethyl-1H-tetrazol-5-ylthiomethyl, 1-sulphomethyl-1H-tetrazol-5-ylthiomethyl, 1-isopropyl-1H-tetrazol-5-ylthiomethyl, 1-(2,2,2-trifluoro)ethyl-1H-tetrazol-5-ylthiomethyl, 1-phenyl-1H-tetrazol-5-ylthiomethyl, 1-(2-methylthio)ethyl-1H-tetrazol-5-ylthiomethyl, 1,3,4-thiadiazol-2-ylthiomethyl, 5-methyl-1,3,4-thiadiazol-2-ylthiomethyl, 1,2,3-thiadiazol-5-ylthiomethyl, 1H-1,2,3-triazol-4-ylthiomethyl, 5-trifluoromethyl-1H-1,2,4-triazol-3-ylthiomethyl, 4,6-dimethylpyrimid-2-ylthiomethyl, 2-thiazolin-2-ylthiomethyl, benzoxazol-2-ylthiomethyl, benzthiazol-2-ylthiomethyl, 2-carboxyphenylthiomethyl, (6-carboxymethyl-7-hydroxypyrrolo-[1,2-b]pyridazin-2-yl)thiomethyl, methoxymethyl, hydroxymethyl, azidomethyl, aminomethyl, benzoyloxymethyl, acetylaminomethyl, carbamoyloxymethyl, 2-methylthio-1,3,4-thiadiazol-5-ylthiomethyl, 2-mercapto-1,3,4-thiadiazol-5-ylthiomethyl, 2-acetylamino-1,3,4-thiadiazol-5-ylthiomethyl, 5-methyl-1,2,4-thiadiazol-2-ylthiomethyl, 2-sulphomethyl-1,2,4-oxadiazol-5-ylthiomethyl, 4-methyl-5-(3-carboxypropyl)thiazol-2-ylthiomethyl, 2H-2-methyl-1,2,3-triazol-4-ylthiomethyl, 1H-1,2,4-triazol-2-ylthiomethyl, 4,5-dihydro-6-hydroxy-4-methyl-5-oxo-1,2,4-triazin-3-ylthiomethyl, 2,5-dihydro-6-hydroxy-2-methyl-5-oxo-1,2,4-triazin-3-ylthiomethyl, 1-oxidopyrid-2-ylthiomethyl, imidazo[4,5-b]pyrid-2-ylthiomethyl or imidazo[4,5-b]pyrimidin-2-ylthiomethyl.

4. $R^1$ is 1-methyl-1H-tetrazol-5-ylthiomethyl.

5. $R^2$ is carboxy.

6. $X^2$ is nitrogen.

7. $R^3$ is hydrogen.

8. $R^4$ is hydrogen.

9. $R^5$ is of the formula II in which $R^7$ is of the formula III or IV in which $R^8$ or $R^9$ is 1H-tetrazol-5-yl, 1H-1,2,3-triazol-4-yl or 1H-1,2,4-triazol-3-yl, each optionally substituted on nitrogen by methyl.

Particular compounds of the invention are described in the Examples. The following is a group of preferred compounds:-

7-(4-[3-(3-cyano-2-methylisothioureido)propyl]imidazol-2-yl)amino-3-(1-methyl-1H-tetrazol-5-yl)thiomethylceph-3-em-4-carboxylic acid (Example 4);

7-(4-[3-(1-methyl-1H-tetrazol-5-ylthio)propyl]imidazol-2-yl)amino-3-(1-methyl-1H-tetrazol-5-yl)thiomethylceph-3-em-4-carboxylic acid (Example 11);

7-(4-[3-(1-methyl-1H-tetrazol-5-ylsulphonylamino)-propyl]imidazol-2-yl)amino-3-(1-methyl-1H-tetrazol-5-yl)thiomethylceph-3-em-4-carboxylic acid (Example 13).

A suitable acid-addition salt of the cephalosporin derivative of the invention is, for example, a salt formed with hydrochloric, hydrobromic, phosphoric, sulphuric, citric or maleic acid. A suitable base-addition salt of the cephalosporin derivative of the invention is, for example, an alkali metal salt (e.g. a sodium or potassium salt), an alkaline earth metal

salt (e.g. a calcium or magensium salt), or a salt with a primary, secondary or tertiary organic amine (e.g. triethylamine, procaine, dibenzylamine and N,$N^1$-dibenzylethylenediamine, and other amines which have been used to form salts with cephalosporins).

The cephalosporin derivative of the formula I may be manufactured by methods known in themselves for the manufacture of chemically analogous compounds. The following processes, $R^0$, $R^1$, $R^2$, $R^3$, $R^4$, $R^5$, $X^1$ and $X^2$ having the meanings stated above, unless indicated otherwise, are therefore provided as further features of the invention.

The process of the invention is characterised by:-

(a) for those compounds in which $R^2$ is carboxy or a heterocyclic radical carrying an acidic proton, and there is optionally carboxy in another part of the molecule, deprotection of the corresponding compound which carries a protecting group, or groups, in place of the acidic hydrogen atom, or atoms. When $R^2$ is carboxy a particularly useful protecting group is diphenylmethyl or p-methoxybenzyl. Such a protecting group may be removed by treatment with a strong organic acid, for example trifluoroacetic acid. A further particularly useful protecting group is t-butyl. This protecting group may be removed by treatment with a strong organic acid such as trifluoroacetic or formic acid. The process may be conducted in the presence of excess organic acid as diluent or solvent or in the presence of an additional diluent or solvent such as anisole or toluene. The process is preferably conducted at or below ambient temperature and preferably over a period of from 5 minutes to 5 hours. Other useful protecting groups are trimethylsilyl (removed by water), benzyl and substituted benzyl, for example

p-nitrobenzyl or p-methoxybenzyl (removed by hydrogenolysis) and 2,2,2-trichloroethyl (removed by zinc/acetic acid).

(b) reaction of a compound of the formula XXX:-

[Formula XXX]

with a compound of the formula XXXI:-

[Formula XXXI]

in which $R^{45}$ is a displaceable radical. $R^{45}$ is for example halogen, preferably fluorine or chlorine. The reaction is preferably conducted in the presence of at least one equivalent of an acid in order that the compound of the formula XXXI is in the protonated form. The reaction may be conducted in the presence of a diluent or solvent, for example acetonitrile, dimethylformamide or tetrahydrofuran or mixtures of these and it may be accelerated or completed by the application of heat, for example by heating to $85^o$ or to the boiling point of the diluent or solvent. The compound of the formula XXXI may conveniently be prepared in situ by prior reaction of the corresponding N-triphenylmethyl derivative with toluene-p-sulphonic acid. The compound of the formula XXX is then added to the reaction mixture.

(c) for those compounds in which $R^1$ is a radical of the formula $CH_2Y$, reaction of a compound of the formula I in which $R^1$ is a radical of the formula $CH_2-R^{45}$ in which $R^{45}$ is a displaceable radical with a compound of the formula Y-H. $R^{45}$ is, for example, halogen or acetoxymethyl. The reaction may be conducted in a diluent or solvent such as acetonitrile, in the presence of boron trifluoride. The reaction may be accelerated or completed by the application of heat, for example by heating to $50^o$.

(d) for those compounds in which $R^8$ or $R^9$ is a radical which contains an NH group, deprotection of the corresponding compound which carries a protecting group in place of the hydrogen atom of the NH group. A suitable protecting group is one of those used in peptide chemistry, for example benzyloxycarbonyl, p-nitrobenzyloxycarbonyl, t-butoxycarbonyl or allyloxycarbonyl. Such a group may be removed by the standard methods used in peptide chemistry.

(e) for those compound in which $R^3$ is other than hydrogen, and $X^2$ is nitrogen, reaction of a compound for the formula XXX with a compound of the formula XXXII:-

[Formula XXXII]

in which $R^{46}$ has the value given above for $R^3$ other than hydrogen, $R^{47}$ is a displaceable radical and $Z^{\ominus}$ is an anion. $R^{47}$ is, for example, 1-6C alkoxy or 1-6C alkylthio for example methoxy or methylthio. $Z^{\ominus}$ is, for example, a halide anion, for example a chloride, bromide or iodide or a methanesulphonate or toluene-p-sulphonate. The reaction may be conducted in a diluent or solvent such as methanol, water or an aqueous buffer. It is preferably conducted at ambient temperature.

When the process of the invention manufactures the compound of the formula I in the form of the free acid or free base, or the zwitterion, and a salt is required, the compound of the formula I in the free acid or zwitterionic form is reacted with a base which affords a pharmaceutically-acceptable cation, or the compound of the formula I in the free base or zwitterionic form is reacted with an acid which affords a pharmaceutically-acceptable anion. When the process of the invention manufactures the compound of the formula I in the form of an acid-addition salt and the zwitterionic form is required, the compound of the

formula I in the form of the acid-addition salt is reacted with a low molecular weight epoxide such as epoxypropane.

The starting material for use in process (a) may be prepared using one of the processes (b) to (e) inclusive of the invention in which the acidic radical is in the protected form. Use of process (b) is illustrated in Examples 5 and 20.

Many of the starting materials of the formula XXX for use in process (b) are described in European Patent Publications Nos. 31708 and 55562. A large number of starting materials of the formula XXX are also known in the cephalosporin art. Novel starting materials of the formula XXXI may be prepared from known compounds, by standard chemical reactions, for example as illustrated in Examples 1-15, and 17-20.

As noted above the cephalosporin derivatives of the invention have antibacterial properties. Thus they are useful antibacterial agents, many of them having a broad spectrum of activity in vitro against standard laboratory microorganisms, both Gram-negative and Gram-positive, which are used to screen for activity against pathogenic bacteria. The antibacterial spectrum and potency of a particular compound may be determined in a standard test system.

The antibacterial properties of the compounds of the invention may also be demonstrated in conventional mouse protection tests.

Cephalosporin derivatives have generally been found to be relatively non-toxic to warm-blooded animals, and this generalisation holds true for the compounds of the present invention. Thus the compound 7-(4-[3-(3-cyano-2-methylisothioureido)propyl]imidazol-2-yl)amino-3-(1-methyl-1H-tetrazol-5-yl)thiomethylceph-3-em-4-carboxylic acid (Example 4) was administered subcutaneously to mice at a dose four times that of the

minimum effective dose which protected 50% of the mice against infection with Salmonella dublin. No overt toxic symptoms or side effects attributable to the administered compound were noted.

The results set out in the following Table are illustrative of the biological activity of one of the preferred compounds of the present invention, that of Example 4. The following results are those obtained on a standard in vitro test system using Jewell and Pearmain agar medium. The antibacterial activity is described in terms of the minimum inhibitory concentration (MIC) determined by agar - dilution technique with an inoculum size of ~ $10^5$ CFU.

| Organism | Code | MIC µg/ml |
|---|---|---|
| Strep. pyogenes | A1 | 8 |
| Staph. aureus | A6 | 16 |
| E. coli | A8 | 0.06 |
| Salmonella dublin | A20 | 0.03 |
| K. aerogenes | A10 | 0.12 |
| Ent. cloacae | A13 | 32 |
| Serratia marescens | A16 | 4 |
| Proteus mirabilis | A18 | 1 |
| Ps. aeruginosa | A21 | >128 |

According to a further feature of the invention there is provided a pharmaceutical composition which comprises a cephalosporin derivative of the invention in association with a non-toxic pharmaceutically-acceptable diluent or carrier.

The pharmaceutical composition of the invention may, for example, be in a form suitable for oral, rectal or parenteral administration, for which purposes it may be formulated by means known to the art into the form of, for example, tablets, capsules, aqueous or oily solutions or suspensions, emulsions, dispersible powders, suppositories and sterile injectable aqueous or oily solutions or suspensions.

In addition to the cephalosporin derivative of the formula I the pharmaceutical composition of the invention may also contain, or be co-administered with, one or more known drugs selected from other clinically useful antibacterial agents (for example other β-lactams or aminoglycosides), inhibitors of β-lactamase (for example clavulanic acid), renal tubular blocking agents (e.g. probenicid) and inhibitors of metabolising enzymes (for example inhibitors of peptidases, for example Z-2-acylamino-3-substituted propenoates).

A preferred pharmaceutical composition of the invention is one suitable for intravenous, subcutaneous or intramuscular injection, for example a sterile injectable contaning between 1 and 10% w/w of the cephalosporin derivative, or one suitable for oral administration in unit dosage form, for example a tablet or capsule which contains between 100 mg. and 1 g. of the cephalosporin derivative.

The pharmaceutical composition of the invention will normally be administered to man in order to combat infections caused by bacteria, in the same general manner as that employed for cephalothin, cefoxitin,

cephradine and other known clinically used cephalosporin derivatives, due allowance being made in terms of dose levels for the potency of the cephalosporin derivative of the present invetion relative to the known clinically used cephalosporins. Thus each patient will receive a daily intravenous, subcutaneous or intramuscular dose of 0.5 to 50 g., and preferably 0.5 to 10 g., of the cephalosporin derivative, the composition being administered 1 to 4 times per day. The intravenous, subcutaneous and intramuscular dose will be given by means of a bolus injection. Alternatively the intravenous dose may be given by continuous infusion over a period of time. Alternatively each patient will receive a daily oral dose which is approximately equivalent to the daily parenteral dose. Thus a preferred daily oral dose is 0.5 to 10 g. of the cephalosporin derivative, the composition being administered 1 to 4 times per day.

The invention is illustrated, but not limited, by the following Examples. The n.m.r. spectra are quoted in $\delta$ relative to tetramethylsilane ($\delta$ = 0) as internal standard, (s = singlet, d = doublet, t = triplet, q = quartet, m = multiplet, br = broad). The temperatures are in degrees Centigrade. The following contractions are used:-

| | | |
|---|---|---|
| TFA | = | trifluoroacetic acid |
| THF | = | tetrahydrofuran |
| HOAc | = | acetic acid |
| EtOAc | = | ethyl acetate |
| MeOH | = | methanol |
| DMF | = | dimethylformamide |
| DMSO | = | dimethylsulphoxide |
| ether | = | diethyl ether |
| HPLC | = | high pressure liquid chromatography |

In the examples the cephalosporin derivative of the invention is isolated in the form of a salt, either an internal salt (a zwitterion) or a salt with an acid such as HBr or $CF_3COOH$. The actual salt which is isolated is dependent on a number of factors including the basicity of the product, the reaction work-up and purification conditions used and the nature of the starting material (salt or free base).

Examples 1-4

As a general process, 7-imidazolylamino cephalosporins were prepared as follows.

A solution of a substituted 2-fluoro-1-triphenylmethylimidazole (1 mM) in dry DMF (2 ml.) was treated with toluene-p-sulphonic acid monohydrate (1 mM) and stirred at 85° for 5 minutes, monitoring the loss of the triphenylmethyl group by HPLC. The appropriate 7-aminoceph-3-em-4-carboxylic acid (1 mM) was added, and heating continued for 2.5 hours, again monitoring the progress of the reaction by HPLC. The reaction mixture was then poured into the eluant to be used for purification (10 ml.), filtered and purified by preparative HPLC on Whatman "Partisil 10" using the indicated mixture of water/MeOH/HOAc, v/v/v.

Using this process, the following compounds were prepared.

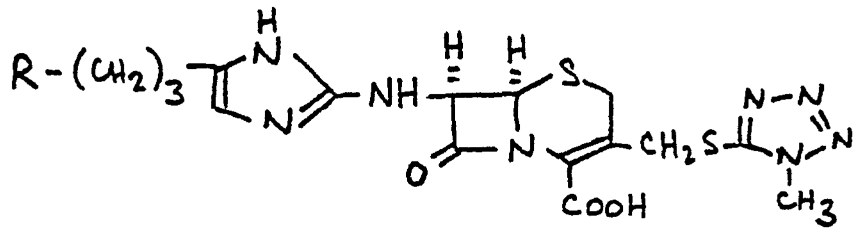

| Example | R- | HPLC eluant |
|---|---|---|
| 1 | 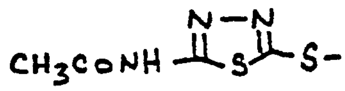 | 50:50:1 |
| 2 | 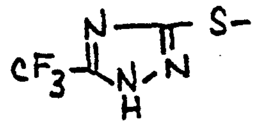 | 50:50:1 |
| 3 | $CF_3SO_2NH-$ | 50:50:1 |
| 4 | 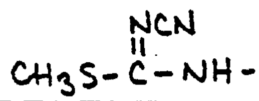 | 55:45:1 |

<u>Notes</u> : The HPLC eluant was the indicated mixture of water/MeOH/HOAc v/v/v. The n.m.r. spectra, obtained in $d_6$DMSO + $CD_3CO_2D$, were as follows:-

Example 1 : 1.9 (m, 2H); 2.1 (s, 3H); 2.43 (t, 2H); 3.15 (t, 2H); 3.53 (q, 2H); 3.85 (s, 3H); 4.24 (br s, 2H); 4.98 (d, 1H); 5.44 (d, 1H); 6.51 (s, 1H).

Example 2 : 1.9 (m, 2H); 2.45 (t, 2H); 3.15 (t, 2H); 3.65 (q, 2H); 3.86 (s, 3H); 4.24 (br s, 2H); 5.05 (d, 1H); 5.45 (d, 1H); 6.62 (s, 1H).

Example 3 : 1.78 (quintet, 2H); 2.5 (t, 2H); 3.18 (t, 2H); 3.65 (d, 2H); 3.94 (s, 3H); 4.28 (br s, 2H); 5.07 (d, 1H); 5.47 (d, 1H); 6.62 (s, 1H).

Example 4 : 1.8 (m, 2H); 2.4 (m, 2H); 2.5 (s, 3H), 3.3 (t, 2H), 3.62 (q, 2H); 3.91 (s, 3H); 4.3 (br s, 2H); 5.04 (d, 1H); 5.54 (d, 1H); 6.6 (s, 1H).

Starting materials for the above process may be obtained as follows:-

Example 1 : A solution of 2-acetylamino-1,3,4-thiadiazole-5-thiol in DMF was treated with sodium hydride. To this mixture was added 2-fluoro-4-(3-methanesulphonyloxypropyl)-1-triphenylmethylimidazole, and the whole was stirred for 1 hour at 70°. Extractive work-up, followed by chromatography,gave 4-[3-(2-acetylamino-1,3,4-thiadiazol-5-ylthio)propyl]-2-fluoro-1-triphenylmethylimidazole, having the following n.m.r. spectrum in $CDCl_3$ : 2.05 (m, 2H); 2.4 (s, 3H); 2.53 (t, 2H); 3.23 (t, 2H); 6.26 (s, 1H); 7.0-7.5 (m, 15H).

Example 2 : Using the same technique as for the starting material for Example 1, but starting with 3-trifluoromethyl-1,2,4-triazole-5-thiol, there was obtained 2-fluoro-4-[3-(3-trifluoromethyl-1,2,4-triazol-5-ylthio)-propyl]-1-triphenylmethylimidazole, having the following n.m.r. spectrum in $CDCl_3$ : 2.15 (m, 2H); 2.7 (t, 2H); 3.24 (t, 2H); 6.38 (s, 1H); 7.0-7.5 (m, 15H).

Example 3 : 4-(3-Aminopropyl)-2-fluoro-1-triphenylmethylimidazole was treated with trifluoromethanesulphonic anhydride to give 2-fluoro-4-(3-trifluoromethanesulphonylaminopropyl)-1-triphenylmethylimidazole, having the following n.m.r. spectrum in $CDCl_3$ : 1.81 (quintet, 2H); 2.5 (t, 2H); 3.31 (t, 2H); 6.23 (s, 1H); 6.95-7.45 (m, 15H).

Example 4 : 4-(3-Aminopropyl)-2-fluoro-1-triphenylmethylimidazole was reacted overnight in EtOH with dimethyl (cyanimido)dithiocarbonate, to give, after extractive work-up and chromatography, 4-[3-(3-cyano-2-methylisothioureido)propyl]-2-fluoro-1-triphenylmethylimidazole, having the following n.m.r. spectrum in $CDCl_3$ : 1.9 (quintet, 2H); 2.47 (s, 3H); 2.5 (m, 2H); 3.45 (q, 2H); 6.28 (s, 1H); 7.0-7.35 (m, 15H); 7.9

(m, 1H).

Example 5

A solution of t-butyl 3-acetoxymethyl-7-(4-[3-(1,2,3-triazol-5-ylthio)propyl]imidazol-2-yl)-aminoceph-3-em-4-carboxylate in TFA at 0° was stirred for 2 hours and then evaporated to dryness. The residue was purified by trituration with ether to give 7-(4-[3-(1,2,3-triazol-5-ylthio)propyl]imidazol-2-yl)-aminoceph-3-em-4-carboxylic acid, having the following n.m.r. spectrum in $d_6$DMSO + $CD_3CO_2D$ : 1.72 (m, 2H); 2.0 (s, 3H); 2.54 (t, 2H); 2.87 (t, 2H); 3.55 (q, 2H); 4.83 (q, 2H); 5.14 (d, 1H); 5.52 (d, 1H); 6.71 (s, 1H).

The starting material for the above process may be obtained as follows:-

A solution of 2-fluoro-4-(3-methanesulphonyl-oxypropyl)-1-triphenylmethylimidazole in 1,2-dimethoxyethane was treated with the sodium salt of 1,2,3-triazole-5-thiol, and the mixture stirred for 2 hours at 70°. Extractive work-up, followed by chromatography, gave 2-fluoro-4-[3-(1,2,3-triazol-5-ylthio)propyl]-1-triphenylmethylimidazole. This imidazole was condensed with t-butyl 3-acetoxymethyl-7-aminoceph-3-em-4-carboxylate, to give t-butyl 3-acetoxymethyl-7-(4-[3-(1,2,3-triazol-5-ylthio)propyl]imidazol-2-yl)aminoceph-3-em-4-carboxylate, having the following n.m.r. spectrum in $CD_3OD$ : 1.56 (s, 9H); 1.79 (m, 2H); 2.07 (s, 3H); 2.52 (t, 2H); 2.89 (t, 2H); 3.53 (q, 2H); 4.84 (q, 2H); 5.14 (d, 1H); 5.51 (d, 1H); 6.32 (s, 1H).

Examples 6-7

The process of Example 1 was repeated, using the appropriate starting materials, to give the following compounds:

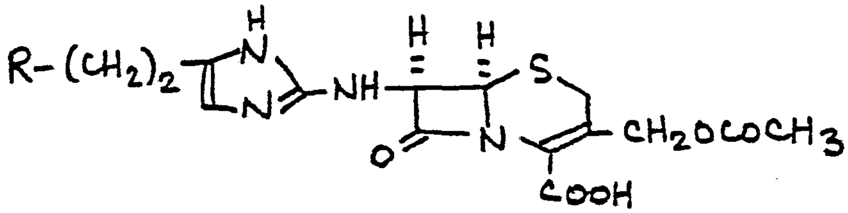

| Example | R− | HPLC eluant |
|---|---|---|
| 6 | 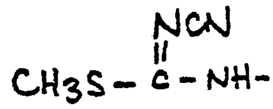 | 50:50:1 |
| 7 | 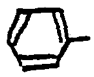 $-CH_2OCONHCH_2CONH-$ | 50:50:1 |

Notes : The HPLC eluant was the indicated mixture of water/MeOH/HOAc v/v/v. The n.m.r. spectra, obtained in $d_6$DMSO + $CD_3CO_2D$, were as follows:

Example 6 : 1.82 (s, 3H); 2.34 (s, 3H); 2.48 (t, 2H); 3.34 (m, 4H); 4.69 (q, 2H); 4.93 (d, 1H); 5.4 (d, 1H); 6.43 (s, 1H).

Example 7 : 1.92 (s, 3H); 2.46 (m, 2H); 3.2 (m, 2H); 3.5 (br s, 2H); 4.78 (q, 2H); 4.94 (s, 2H); 5.02 (d, 1H); 5.47 (d, 1H); 6.5 (s, 1H); 7.22 (s, 5H).

Starting materials for the above process may be obtained as follows:-

Example 6 : 4-(2-Aminoethyl)-2-fluoro-1-triphenylmethylimidazole was treated with dimethyl (cyanimido)dithiocarbonate in MeOH for 18 hours at 20°. Chromatographic work-up gave 4-[2-(3-cyano-2-methylisothioureido)-ethyl]-2-fluoro-1-triphenylmethylimidazole, having the following n.m.r. spectrum in $CDCl_3$ : 2.45 (s, 3H); 2.6 (t, 2H); 3.55 (q, 2H); 6.3 (s, 1H); 7.0-7.3 (m, 15H); 7.7 (m, 1H).

Example 7 : N-Benzoyloxycarbonylglycine and 4-(2-aminoethyl)-2-fluoro-1-triphenylmethylimidazole were condensed by the mixed anhydride procedure to give 4-[2-(benzyloxycarbonylaminoacetylamino)ethyl]-2-fluoro-1-triphenylmethylimidazole, which was used without further characterisation.

Examples 8-11

The process described in Example 1 was repeated, using the appropriate starting materials, to give the following compounds.

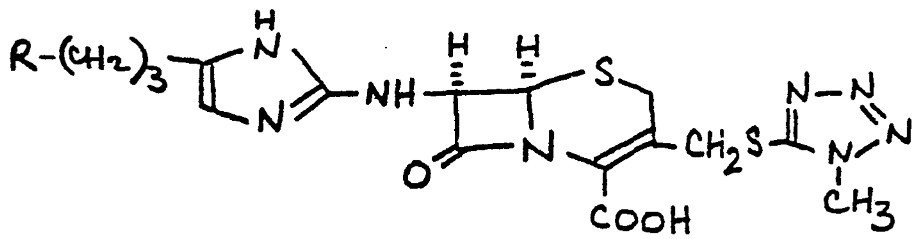

| Example | R- | HPLC eluant |
|---|---|---|
| 8 | 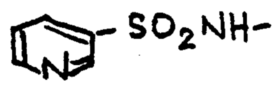 | 65:35:1 |
| 9 | 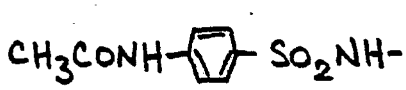 | 63:37:1 |
| 10 | 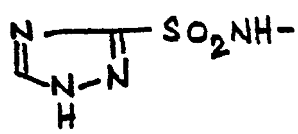 | 75:25:1 |
| 11 | 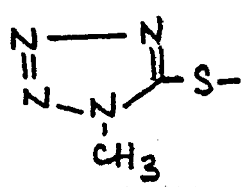 | 60:40:1 |

Notes: The HPLC eluant was the indicated mixture of water/MeOH/HOAc v/v/v. The n.m.r. spectra of the products, obtained in $d_6$DMSO + $CD_3CO_2D$, were as follows.

Example 8: 1.78 (m, 2H); 2.45 (t, 2H); 2.82 (t, 2H); 3.55 (d, 1H); 3.8 (d, 1H); 3.92 (s, 3H); 4.32 (s, 2H); 5.08 (d, 1H); 5.55 (d, 1H), 6.59 (s, 1H); 7.61 (d of d, 1H); 8.16 (d of t, 1H); 8.79 (d, 1H); 8.95 (d, 1H).

Example 9: 1.68 (m, 2H); 2.07 (s, 3H); 2.45 (t, 2H); 2.73 (t, 2H); 3.57 (d, 1H); 3.75 (d, 1H); 3.92 (s, 3H); 4.32 (s, 2H); 5.06 (d, 1H); 5.51 (d, 1H); 6.67 (s, 1H); 7.72 (s, 4H).

Example 10: 1.78 (m, 2H); 2.45 (t, 2H); 3.04 (t, 2H); 3.6 (d, 1H); 3.8 (d, 1H); 3.9 (s, 3H); 4.37 (s, 2H); 5.12 (d, 1H); 5.56 (d, 1H); 6.68 (s, 1H); 8.76 (s, 1H).

Example 11: 2.0 (m, 2H); 2.52 (m, 2H); 3.29 (t, 2H); 3.66 (d, 2H); 3.91 (s, 6H); 4.32 (br s, 2H); 5.09 (d, 1H); 5.51 (d, 1H); 6.66 (s, 1H).

Starting materials for the above process may be obtained as follows:-

Example 8: Equivalent quantities of 4-(3-aminopropyl)-2-fluoro-1-triphenylmethylimidazole and pyridine-3-sulphonyl chloride were reacted in pyridine. Extractive work up, followed by chromatography, gave 2-fluoro-4-[3-(pyrid-3-ylsulphonylamino)propyl]-1-triphenylmethylimidazole, having the following n.m.r. spectrum in $CDCl_3$:- 1.78 (quintet, 2H); 2.45 (t,2H); 3.05 (q, 2H); 5.73 (t, 1H); 6.24 (s, 1H); 7.04-7.45 (m, 16H); 8.13 (d of q, 1H); 8.76 (d of d, 1H); 9.07 (d, 1H).

Example 9: Equivalent quantities of 4-(3-aminopropyl)-2-fluoro-1-triphenylmethylimidazole and 4-acetylaminobenzene-sulphonyl chloride were reacted together in dichloromethane solution in the presence 4-dimethylaminopyridine. Extractive work up gave 4-[3-(4-acetylaminobenzenesulphonyl-amino)propyl]-2-fluoro-1-triphenylmethylimidazole, having the following n.m.r. spectrum in $CDCl_3$:- 1.71 (quintet, 2H); 2.16 (s, 3H); 2.4 (t, 2H); 2.85 (q, 2H); 6.25 (s, 1H); 7.04-7.44 (m, 15H); 7.76 (s, 4H).

Example 10: The same procedure as for the starting material of Example 9 was used with the appropriate sulphonyl chloride to give 2-fluoro-4-[3-(1,2,4-triazol-4-ylsulphonylamino)-propyl]-1-triphenylmethylimidazole, having the following

n.m.r. spectrum in $CDCl_3$:- 1.8 (m, 2H); 2.42 (t, 2H); 3.1 (m, 2H); 6.23 (s, 1H); 6.5 (br, 2H); 7.04-7.44 (m, 15H); 8.26 (s, 1H).

Example 11: A solution of 1-methyl-1H-1,2,3,4-tetrazole-5-thiol in DMF was treated with sodium hydride at room temperature. 2-Fluoro-4-(3-methanesulphonyloxypropyl)-1-triphenylmethylinidazole was added, and the mixture heated 2 hours at 85°. Extractive work up, followed by chromatography, gave 2-fluoro-4-[3-(1-methyl-1H-1,2,3,4-tetrazol-5-ylthio)propyl]-1-triphenylmethylimidazole, having the following n.m.r. spectrum in $CDCl_3$:- 2.07 (quintet, 2H); 2.58 (t, 2H); 3.33 (t, 2H); 3.89 (s, 3H); 6.28 (s, 1H); 7.0-7.4 (m, 15H).

Examples 12-15

The process described in Example 1 was repeated, using the appropriate starting materials, to give the following compounds:

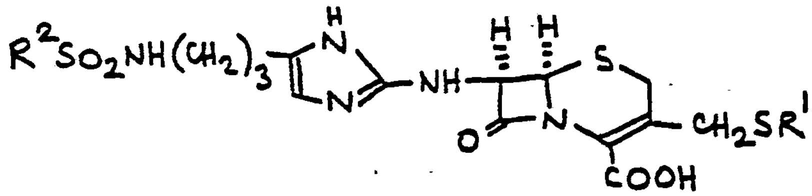

| Example | $-R^2$ | $-R^1$ | HPLC eluant |
|---|---|---|---|
| 12 | 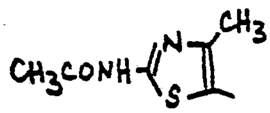 | 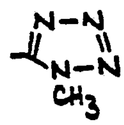 | 55:45:1 |
| 13 | 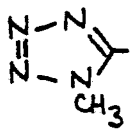 | 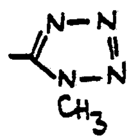 | 60:40:1 |
| 14 | 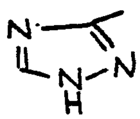 | 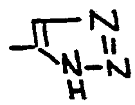 | 75:25:1 |
| 15 | 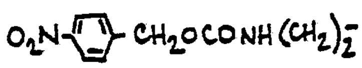 | 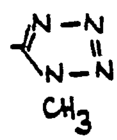 | 50:50:1 |

Notes: The HPLC eluant was the indicated mixture of water/MeOH/HOAc v/v/v. The n.m.r. spectra of the products, obtained in $d_6$DMSO + $CD_3CO_2D$, were as follows.

Example 12 : 1.67 (m, 2H); 2.13 (s, 3H); 2.41 (s, 3H); 2.47 (m, 2H); 2.84 (t, 2H); 3.48 (d, 1H); 3.67 (d, 1H); 3.91 (s, 3H); 4.28 (d, 1H); 4.32 (d, 1H); 5.12 (d, 1H); 5.46 (d, 1H); 6.49 (s, 1H).

Example 13 : 1.68 (m, 2H); 2.4 (m, 2H); 3.07 (t, 2H); 3.48 (d, 1H); 3.7 (d, 1H); 3.88 (s, 3H); 4.18 (s, 3H); 4.26 (s, 2H); 5.01 (d, 1H); 5.46 (d, 1H); 6.53 (s, 1H).

Example 14 : 1.7 (m, 2H); 2.4 (m, 2H); 3.0 (t, 2H); 3.5 (d, 1H); 3.72 (d, 1H); 4.03 (s, 2H); 5.1 (d, 1H); 5.48 (d, 1H); 6.58 (s, 1H); 7.88 (s, 1H); 8.72 (s, 1H).

Example 15 : 1.7 (m, 2H); 2.4 (m, 2H); 3.0 (t, 2H); 3.2 (m, 2H); 3.38 (m, 2H); 3.5 (d, 1H); 3.8 (d, 1H); 3.92 (s, 3H); 4.32 (s, 2H); 5.08 (d, 1H); 5.19 (s, 2H); 5.53 (d, 1H); 6.55 (s, 1H); 7.58 (d, 2H); 8.22 (d, 2H).

Starting materials for the above process may be obtained as follows:-

Example 12 : The same procedure as for the starting material of Example 9 was followed, using the appropriate sulphonyl chloride, to give 4-[3-(2-acetylamino-4-methylthiazol-5-ylsulphonylamino)propyl]-2-fluoro-1-triphenylmethylimidazole, having the following n.m.r. spectrum in $CDCl_3$:-
1.78 (m, 2H); 2.2 (s, 3H); 2.45 (t, 2H); 2.46 (s, 3H); 3.04 (t, 2H); 6.22 (s, 1H); 7.04-7.4 (m, 15H).

Example 13 : The same procedure as for the starting material of Example 9 was followed, using the appropriate sulphonyl chloride, to give, after chromatographic work-up, 2-fluoro-4-[3-(1H-1-methyltetrazol-5-ylsulphonylamino)propyl]-1-triphenylmethylimidazole, having the following n.m.r. spectrum in $CDCl_3$:-
1.92 (m, 2H); 2.62 (t, 2H); 3.47 (t, 2H); 4.3 (s, 3H); 6.3 (s, 1H); 7.1-7.5 (m, 15H); 7.9 (br, 1H).

Example 15 : 2-(4-Nitrobenzyloxycarbonylamino)-ethanethiol was suspended in 2N aqueous hydrochloric acid, cooled to -8$^{o}$ and chlorine gas passed in for 45 minutes. The precipitate of 2-(4-nitrobenzyloxycarbonyl-amino)ethanesulphonyl chloride was then subjected to the same procedure as for the starting material of Example 9, followed by chromatography to give 2-fluoro-4-(3-[2-(4-nitrobenzyloxycarbonylamino)ethane-sulphonylamino]propyl)-1-triphenylmethylimidazole, having the following n.m.r. spectrum in $CDCl_3$:- 1.77 (m, 2H); 2.47 (t, 2H); 3.1 (m, 2H); 3.15 (m, 2H); 3.58 (m, 2H); 5.13 (s, 2H); 6.23 (s, 1H); 7.0-7.4 (m, 15H); 7.4 (d, 2H); 8.15 (d, 2H)

Example 16

The product of Example 15 was dissolved in ethanol containing a trace of 2N aqueous hydrochloric acid, treated with a 50% weight excess of 10% w/w palladium on charcoal, and hydrogenated for 3 hours. After filtration, the product was purified according to the procedure of Example 1 using 80:20:1 v/v/v water/MeOH/HOAc as HPLC eluant, to give 7-(4-[3-(2-aminoethanesulphonylamino)propyl]imidazol-2-yl)amino-3-(1H-1-methyltetrazol-5-yl)thiomethylceph-3-em-4-carboxylic acid, having the following n.m.r. spectrum in $d_6$DMSO + $CD_3CO_2D$:- 1.78 (m, 2H); 2.44 (m, 2H); 3.03 (m, 2H); 3.2-3.5 (m, 4H); 3.63 (d, 2H); 3.95 (s, 3H); 4.35 (s, 2H); 5.07 (d, 1H); 5.53 (d, 1H); 6.56 (s, 1H).

Example 17

The process described in Example 1 was repeated, using the appropriate starting material, to give the following compound:

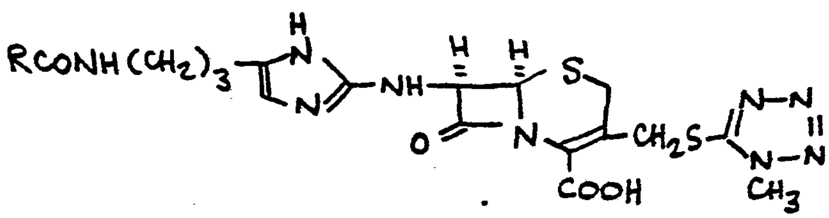

| Example | R | HPLC eluant |
|---|---|---|
| 17 | $CH_2$=CHC$H_2$O.CONHCH-<br>$CH_3$ | 65:35:1 |

Notes: The HPLC eluant was the indicated mixture of water/MeOH/HOAc v/v/v. The n.m.r. spectrum of the product, obtained in $d_6$DMSO + $CD_3CO_2D$, was as follows:

Example 17 : 1.2 (d, 3H); 1.7 (m, 2H); 2.4 (m, 2H); 3.08 (m, 4H); 3.65 (d, 2H); 3.94 (s, 3H); 4.32 (m, 1H); 4.45 (d, 2H); 5-6 (complex, 5H); 6.66 (s, 1H).

Starting material for the above Example may be obtained as follows:

dl-Alanine was dissolved in aqueous 2N sodium hydroxide (2 equivalents) and treated at 0° with allyl chloroformate in toluene. The two phase system was stirred overnight, the aqueous layer acidified, extracted with ether, and N-allyloxycarbonylalanine precipitated as its dicyclohexylamine salt. This salt was dissolved in DMF, cooled to -30°, and treated successively with pivaloyl chloride and 4-(3-aminopropyl)-2-fluoro-1-triphenylmethylimidazole. After extractive work-up and chromatography there was obtained dl-4-[3-(N-allyloxycarbonylalanyl)aminopropyl]-2-fluoro-1-triphenylmethylimidazole, having the following n.m.r. spectrum in $CDCl_3$:- 1.25 (d, 3H); 1.66 (quintet, 2H); 2.34 (t, 2H); 3.16 (q, 2H); 4.1 (m, 1H); 4.42 (d, 2H); 5.05 (m, 1H); 5.25 (m, 1H); 5.49 (d, 1H); 5.6-6.0 (m, 1H); 6.13 (s, 1H); 6.77 (t, 1H); 7.0-7.4 (m, 15H).

Examples 18 and 19

Using the general procedure of Example 1, 7-amino-3-(1H-1-methyltetrazol-5-yl)thiomethylceph-3-em-4-carboxylic acid was reacted with (-)-4-(3-[N-(t-butoxycarbonyl)alanyl]aminopropyl)-2-fluoro-1-triphenylmethylimidazole. The crude product was purified by preparative HPLC, using a gradient elution profile from 90:10:1 to 20:80:1, v/v/v, water/MeOH/HOAc over 30 minutes. The two major peaks were collected.

The first, eluting at 50·50·1 water/MeOH/HOAc, was identified as the toluene-p-sulphonate salt of 7-[4-(3-alanylaminopropyl)imidazol-2-yl]amino-3-(1H-1-methyltetrazol-5-yl)thiomethylceph-3-em-4-carboxylic acid, having the following n.m.r. spectrum in $d_6$DMSO + $CD_3CO_2D$:- 1.36 (d, 3H); 1.74 (m, 2H); 2.4 (m, 2H); 3.16 (m, 2H); 3.75 (d, 2H); 3.84 (m, 1H); 3.93 (s, 3H); 4.32 (s, 2H); 5.14 (d, 1H); 5.53 (d, 1H); 6.72 (s, 1H); plus toluene-p-sulphonic acid at 2.28 (s, 3H); 7.13 (d, 2H); 7.52 (d, 2H).

The second peak, eluting at 30:70:1 water/MeOH/HOAc, was identified as 7-(4-[3-(N-[t-butoxycarbonyl]alanyl)aminopropyl]imidazol-2-yl)amino-3-(1H-1-methyltetrazol-5-yl)thiomethylceph-3-em-4-carboxylic acid, having the following n.m.r. spectrum in $d_6$DMSO + $CD_3CO_2D$:- 1.15 (d, 3H); 1.37 (s, 9H); 1.69 (m, 2H); 2.37 (m, 2H); 3.07 (m, 2H); 3.62 (s, 2H); 3.83 (m, 1H); 3.93 (s, 3H); 4.29 (s, 2H); 5.03 (d, 1H); 5.5 (d, 1H); 6.58 (s, 1H).

Starting material for the above Examples may be prepared as follows:- 4-(3-Aminopropyl)-2-fluoro-1-triphenylmethylimidazole, (-)-N-(t-butoxycarbonyl)-alanine and dicyclohexylcarbodiimide were stirred together at 20° in DMF for 16 hours. EtOAc was added to precipitate dicyclohexylurea, which was filtered off. Chromatography of the filtrate gave (-)-4-(3-[N-(t-butoxycarbonyl)alanyl]aminopropyl)-2-fluoro-1-triphenylmethylimidazole, having the following n.m.r. spectrum in $d_6$DMSO + $CD_3CO_2D$:- 1.19 (d, 3H); 1.41 (s, 9H); 1.68 (m, 2H); 2.38 (t, 2H); 3.08 (m, 2H); 3.95 (m, 1H); 6.34 (s, 1H); 7.1-7.5 (m, 15H).

Example 20

Using the procedure of Example 5 the appropriate t-butyl ester starting material was hydrolysed, and product precipitated from acetone with

ether, to give 3-acetoxymethyl-7-(4-[3-(1-methylthio-2-nitroethenylamino)propyl]imidazol-2-yl)aminoceph-3-em-4-carboxylic acid, having the following n.m.r. spectrum in $d_6$DMSO + $CD_3CO_2D$:- 1.8 (m, 2H); 2.03 (s, 3H); 2.4 (m, 2H); 3.3-3.6 (m, 4H); 4.73 (d, 1H); 5.03 (d, 1H); 5.15 (d, 1H); 5.59 (d, 1H); 6.58 (s, 1H); 6.63 (s, 1H).

The starting material may be obtained as follows:-

A solution of 2,2-bismethylthio-1-nitroethylene and 4-(3-aminopropyl)-2-fluoro-1-triphenylmethylimidazole in dry THF was left to react at 20° for 48 hours. Work up by chromatography gave 2-fluoro-4-[3-(1-methylthio-2-nitroethenylamino)propyl]-1-triphenylmethylimidazole. This imidazole was condensed with t-butyl 3-acetoxymethyl-7-aminoceph-3-em-4-carboxylate in acetonitrile in the presence of toluene-p-sulphonic acid to give, after silica chromatography, t-butyl 3-acetoxymethyl-7-(4-[3-(1-methylthio-2-nitroethenylamino)propyl]imidazol-2-yl)aminoceph-3-em-4-carboxylate, having the following n.m.r. spectrum in $d_6$DMSO + $CD_3CO_2D$:- 1.49 (s, 9H); 1.75 (m, 2H); 2.05 (s, 3H); 2.45 (m, 2H); 2.51 (s, 3H); 3.47 (t, 2H); 3.60 (d, 2H); 4.67 (d, 1H); 4.98 (d, 1H); 5.21 (d, 1H); 5.66 (d, 1H); 6.61 (s, 1H); 6.67 (s, 1H).

Claims

1. A cephalosporin derivative of the formula I:-

[Formula I - given hereafter]

in which $R^0$ is hydrogen or methyl; $R^1$ is any one of the C-3 substituents from antibacterially-active cephalosporins known in the art; $R^2$ is any one of the C-4 substituents from antibacterially-active cephalosporins known in the art; $X^1$ is sulphur, oxygen or $CH_2$; $X^2$ is nitrogen or of the formula $\overset{\oplus}{N}-R^6$; $R^3$ and $R^6$, which may be the same or different, are hydrogen 1-6C alkyl, 1-6C alkanoyl, hydroxy, 1-6C alkoxy, amino, 1-6C alkanoylamino, 1-6C alkylamino, 1-6C aminoalkyl, 1-6C hydroxyalkyl, 2-6C carboxyalkyl, 2-6C alkenyl, 3-6C alkoxyalkyl, 3-8C alkoxycarbonylalkyl, furylmethyl, phenyl, 7-11C phenylalkyl,

in the latter two of which the phenyl ring is optionally substituted by halogen, methyl, methoxy, nitro, hydroxy, amino, carboxy or methoxycarbonyl,

3-6C alkynyl, 3-6C alkadienyl or 9-16C arylalkenyl; one of $R^4$ and $R^5$ is hydrogen, 1-6C alkyl or 7-11C aralkyl and the other is of the formula II:-

[Formula II]

in which n is 1 to 6 and $-R^7$ is of the formula III:-

[Formula III]

in which $R^8$ is 2-6C hydroxyalkyl, 2-6C aminoalkyl, 3-10C alkylaminoalkyl, 4-15C dialkylaminoalkyl (or the N-oxide thereof), 2-6C cyanoalkyl, 3-6C (amino)-(carboxy)alkyl or heteroaryl, or $-R^7$ is of the formula IV:-

[Formula IV]

in which $R^9$ is 2-6C aminoalkyl, p-nitrobenzyloxycarbonyl(2-6C aminoalkyl), 2-8C alkoxyalkyl, 3-10C alkoxycarbonylalkyl, 2-6C alkenyl, 1-6C haloalkyl, 2-6C carbamoylalkyl, 3-10C alkylcarbamoylalkyl, 4-15C dialkylcarbamoylalkyl, 2-6C cyanoalkyl, 6-10C aryl or heteroaryl,
or $-R^7$ is of the formula V:-

[Formula V]

in which $R^{10}$ is 6-10C aryl, furyl, thienyl, thiazolyl or thiadiazolyl, the latter two being optionally substituted by amino, and $R^{11}$ is hydrogen, 1-6C alkyl, 2-8C carboxyalkyl or 3-10C alkoxycarbonylalkyl,
or $-R^7$ is of the formula VI:-

[Formula VI]

in which $R^{12}$ is an acyl radical derived from an aminoacid or from two or three aminoacids linked via normal peptide bonds, the aminoacids (apart from glycine) being in either the D or L configuration and being selected from glycine, alanine, β-alanine, valine, leucine, isoleucine, serine, threonine, arginine, lysine, histidine, aspartic acid, glutamic acid, asparagine, glutamine, cysteine, methionine, phenylalanine, tyrosine, tryptophan and proline, the amino groups in such acyl radicals optionally carrying one or (where possible) more protecting groups selected from benzyloxycarbonyl, p-nitrobenzyloxycarbonyl, t-butoxycarbonyl and allyloxycarbonyl, the glutamic and aspartic acid residues optionally being in the form of the 1-6C alkyl esters and the arginine residue optionally being in the form of the nitro derivative,
or $-R^7$ is of the formula VII:-

[Formula VII]

in which -A- is of the formula VIII:-

[Formula VIII]

in which p is 1 or 2, or A is 3,4-dioxocyclobuten-1,2-diyl or of the formula C=Z in which Z is NCN, $NCONH_2$, $NNO_2$, $CHNO_2$ $C(CN)_2$, $NCOR^{14}$, $NCO_2R^{14}$, $SO_2R^{14}$ or $NR^{15}$ in which $R^{14}$ is 1-6C alkyl or 6-10C aryl and $R^{15}$ is 1-6C alkyl and $R^{13}$ is 1-6C alkoxy, 1-6C alkylthio, amino, 1-6C alkylamino or 2-10C dialkylamino;

wherein when $R^8$ or $R^9$ is heteroaryl it is a 5 or 6 membered heterocyclic aromatic ring containing 1,2,3 or 4 hetero atoms selected from oxygen, nitrogen and sulphur, and wherein when $R^8$ or $R^9$ is heteroaryl or $R^9$ is aryl the ring may be optionally substituted by one or two substituents selected from halogen, 1-6C alkyl, hydroxy, 1-6C alkoxy, mercapto, 1-6C alkylthio, carboxy, 2-6C alkoxycarbonyl, carbamoyl, 2-6C alkylcarbamoyl, 3-10C dialkylcarbamoyl, nitro, amino, 1-6C alkylamino, 2-8C dialkylamino, 1-6C alkanoylamino, 2-10C (alkanoyl)(alkyl)amino, cyano, sulphamoyl, 1-6C alkylsulphamoyl, 2-10C dialkylsulphamoyl, 1-6C haloalkyl, 1-6C alkanesulphinyl and 1-6C alkanesulphonyl;

provided that when $R^{10}$ is heteroaryl and m is 0 then n is 2 to 6;

and where the compound of the formula I contains a free basic or acidic group, the pharmaceutically-acceptable acid- and base-addition salts thereof.

2. A cephalosporin derivative of the formula I:-

[Formula I]

in which $R^0$ is hydrogen or methyl; $R^1$ is hydrogen, halogen (e.g. fluorine, chlorine or bromine), hydroxy or amino or a radical selected from:-

(a) 1-6C alkyl, benzyl optionally substituted by fluorine or methoxy, 1-6C haloalkyl, formyl, carboxy, 1-6C alkoxy, 1-6C alkylthio, 1-6C alkylamino, phenylamino, benzylamino, 3-6C cycloalkylamino, cyano, 2-6C alkoxycarbonyl, 2-6C alkanoyl, 3-10C alkoxycarbonylalkyl, 2-6C alkoxycarbonylamino, 2-6C alkylthiocarbonylamino, piperidino, pyrrolidino, morpholino, 2-6C alkanoylamino, ureido, 2-6C alkylureido, 3-8C dialkylureido, 1-6C alkanesulphinyl, 1-6C alkanesulphonyl, heterocyclyl and heterocyclylthio in which the heterocycle is 1,3,4-thiadiazol-2-yl or 1,3,4-oxadiazol-2-yl, each optionally substituted in the 5-position, 1H-tetrazol-5-yl optionally substituted in the 1-position, or 1H-1,2,3-triazol-4-yl optionally substituted in the 1 or 5 position, the optional substituents in each of these heterocycles being 1-6C alkyl, 1-6C sulphoalkyl, 2-6C carboxyalkyl,

1-6C haloalkyl or 3-6C alkylthioalkyl, or pyridazin-3-yl, oxazol-3-yl or thiazol-3-yl each optionally substituted by 1 or 2 radicals selected from 1-6C alkyl, 1-6C haloalkyl and 2-6C alkoxycarbonyl;

(b) radicals of the formula X:-

[Formula X]

in which $R^{16}$ and $R^{17}$, which may be the same or different, are hydrogen, 1-6C alkyl, 5-7C cycloaliphatic, 6-12C aryl, 7-10C arylalkyl (e.g. benzyl, 2-phenethyl), formyl, cyano, carboxy, 2-6C alkoxycarbonyl, sulpho, 1-6C alkanesulphinyl, 1-6C alkanesulphonyl, 1-6C alkoxy, 1-6C alkylthio, carbamoyl, nitro, 1-6C hydroxyalkyl, methylcarbamoyloxymethyl, benzylcarbamoyloxymethyl, 2-6C alkoxymethyl, 2-6C alkylthiomethyl, 2-haloethoxymethyl, cyclopentyloxymethyl, benzyloxymethyl or 3-8C alkanoyloxymethyl or radicals of the formula $CH_2SHet^1$ in which $Het^1$ is 1,3,4-thiadiazol-2-yl or 1,3,4-oxadiazol-2-yl both optionally substituted in the 5-position by methyl, 1H-triazol-5-yl optionally substituted in the 1-position by methyl or 1H-1,2,3-triazol-4-yl;

(c) radicals of the formula XI:-

[Formula XI]

in which $R^{18}$ is cyano, carboxy or 2-6C alkoxycarbonyl;

(d) radicals of the formula XII:-

[Formula XII]

in which $R^{19}$ and $R^{20}$, which may be the same or different, are hydrogen or 1-6C alkyl and e is 1 to 4;

(e) radicals of the formula $CH_2Y$ in which Y is an atom or group which is the residue of a nucleophile or a derivative of a residue of a nucleophile, such a nucleophile or a derivative thereof being:-

A. 3-15C trialkylamines;

B. heterocyclic amines having more than one heteroatom, at least one heteroatom being nitrogen;

C. pyridines which ar optionally substituted by 1 to 3 substituents selected from halogen, 1-6C alkyl, 6-10C aryl, 7-11C arylalkyl, 2-10C alkoxyalkyl, 3-10C alkanoyloxymethyl, formyl, carbamoyl, 2-6C alkanoyloxy, 2-6C alkoxycarbonyl, 1-6C alkoxy, 6-10C aryloxy, 7-11C aralkoxy, 1-6C alkylthio, 6-10C arylthio, 7-11C aralkylthio, cyano, hydroxy, 2-6C alkylcarbamoyl, 3-10C dialkylcarbamoyl, 2-6C (hydroxyalkyl)carbamoyl and 2-6C carbamoylalkyl;

D. azide;

E. amino, 1-6C alkanoylamino and 7-11C aroylamino;

F. cyanide, pyrrole and substituted pyrroles;

G. nucleophiles giving rise to $R^1$ of the formula XIII:-

[Formula XIII]

in which $R^{21}$ and $R^{22}$, which may be the same or different, are selected from hydrogen, cyano, 1-6C alkyl, 2-6C alkoxycarbonyl, 8-20C mono- or di-aryl-alkoxycarbonyl, 2-6C alkanoyl, 7-11C aralkyl, cyclopentyl and cyclohexyl, and phenyl optionally substituted by 1 or 2 radicals selected from halogen, 1-6C alkyl, 1-6C alkoxy, 1-6C alkylamino, nitro and amino, and $R^{23}$ is selected from hydrogen, 1-6C alkyl, 7-11C aralkyl, cyclopentyl and cyclohexyl, and phenyl

optionally substituted by 1 or 2 radicals selected from halogen, 1-6C alkyl, 1-6C alkoxy and 1-6C alkylamino;

H. thiourea optionally substituted by 1-6C alkyl, 6-10C aryl, 5-7C alicyclic or heterocyclyl, dithiocarbamates, thioamides substituted by 1-6C alkyl or 6-10C aryl or thiosemicarbazides, thiosulphates, arylthioacids or heterocyclicthioacids of up to 10 carbons and dithioacids of the formula XIV:-

[Formula XIV]

in which $R^{24}$ and $R^{25}$, which may be the same or different, are hydrogen, 1-6C alkyl, 2-6C hydroxyalkyl, 3-8C alkylaminoalkyl, 4-10C dialkylaminoalkyl or phenyl, or $R^{24}$ and $R^{25}$ are joined to form a pyrrolidine, piperidine or morpholine ring or a piperazine ring which is optionally substituted on nitrogen by one or two (in quaternised form) radicals selected from 1-6C alkyl and 3-6C alkenyl;

I. compounds of the formula $R^{26}S(O)_dH$ in which d is 0, 1 or 2 and $R^{26}$ is 1-6C alkyl, 5-7C alicyclic, 6-10C aryl optionally substituted by carboxy, or 7-11C arylalkyl or a 5- or 6-membered heterocyclic ring (partially or fully unsaturated) containing 1 to 4 nitrogens which ring may further include (where possible) oxygen and/or sulphur, in which the nitrogen may be in the oxide form, which heterocylic ring may be fused with another heterocyclic ring within the same definition or may be fused with a benzene ring, the above aryl, arylalkyl, heterocyclic or fused benzene ring being optionally substituted (where possible) by 1 or 2 substituents selected from halogen, 1-6C alkyl, 1-6C haloalkyl, 6-10C aryl, 2-6C alkenyl, 1-6C alkoxy, oxo, hydroxy, mercapto, amino, carboxy, cyano, isothiocyanate, carbamoyl, sulphamoyl, 2-6C alkoxycarbonyl, 3-6C alkenyloxycarbonyl, 8-12C

aralkylcarbonyl, 7-11C aryloxycarbonyl, 2-6C hydroxyalkyl, 3-6C dihydroxyalkyl, sulphoamino and 1-6C alkanesulphonylamino and radicals of the formula B-$R^{27}$ in which B is a 2-8C straight or branched chain which may be interrupted by sulphur, oxygen, NH or 1-6C N-alkyl and $R^{27}$ is selected from hydroxy, mercapto, cyano, 1-6C alkylamino, 2-6C dialkylamino, 2-6C alkanoylamino, carboxy, sulpho, carbamoyl, sulphamoyl, amidino, guanidino, 2-6C alkoxycarbonyl, 2-6C alkylcarbamoyl, 2-6C dialkylcarbamoyl, 1-6C alkylsulphamoyl, 2-6C dialkylsulphamoyl, sulphoamino, ureido, 1-6C alkoxy, 1-6C alkylthio, 1-6C alkanesulphonyl, 2-6C alkanoyl and 2-6C alkanoyloxy and radicals of the formula -S-$R^{28}$ in which $R^{28}$ is 1-6C alkyl or a group of the formula B-$R^{27}$ in which B and $R^{27}$ have the meanings given above and radials of the formula $NR^{29}R^{30}$ in which $R^{29}$ and $R^{30}$, which may be the same or different, are selected from 1-6C alkyl, groups of the formula B-$R^{27}$ in which B and $R^{27}$ have the definitions given above, 1-6C alkoxycarbonyl, 2-6C alkanoyl, carbamoyl, 2-6C alkylcarbamoyl and 3-10C dialkylcarbamoyl;

J. radicals of the formula $R^{31}$-OH in which $R^{31}$ is hydrogen, 1-6C alkyl, 3-6C alkenyl, 3-6C alkynyl, 5-7C cycloalkyl, 6-12C cycloalkylalkyl, 6-10C aryl, 7-11C arylalkyl or furfuryl, any of which may be substituted by 1 or 2 radicals selected from halogen, 1-6C alkyl, nitro, hydroxy, carboxy, 2-6C alkanoyloxy, 2-6C alkoxycarbonyl, 2-6C alkanoyl, 1-6C alkanesulphonyl, 1-6C alkoxysulphonyl, amino, 1-6C alkylamino and 2-6C alkanoylamino, or $R^{31}$ is carbamoyl;

K. radicals of the formula $R^{32}$-Q-COOH in which Q is a direct bond, oxygen, sulphur or NH and $R^{32}$ is:-

(i) hydrogen or 1-6C alkyl which may be interrupted by oxygen, sulphur or NH or substituted by a cyano, carboxy, methoxycarbonyl, ethoxycarbonyl, hydroxy, carboxycarbonyl, halogen or amino;

(ii) 2-6C alkenyl which may be interrupted by oxygen, sulphur or NH;

(iii) phenyl, hydroxyphenyl, chlorophenyl, fluorophenyl, tolyl, nitrophenyl, aminophenyl, methoxyphenyl, methylthiophenyl, thienyl, pyridyl, cyclohexyl, cyclopentyl, sydnonyl, naphthyl or ethoxynaphthyl;

or (iv) $R^{33}$-$(CH_2)_g$ where $R^{33}$ has the value for $R^{32}$ listed in (i) above and g is 1 to 4;

and (f) radicals of the formula XV:-

[Formula XV]

in which $R^{34}$ is

1) 1-6C alkyl (e.g. methyl), L-2-amino-2-carboxyethyl or phenyl;

2) pyridyl or the N-oxide thereof;

3) pyridazin-3-yl substituted in the 6-position by 1-6C alkyl (e.g. methyl), methoxy, amino or 1-6C acylamino (e.g. acetylamino), or the N-oxide thereof, or pyrimidin-2-yl or tetrazolo[4,5-b]pyridazin-6-yl;

4) 5,6-dioxo-1,4,5,6-tetrahydro-1,2,4-triazin-3-yl substituted in the 4-position; 1,3,4-triazol-5-yl or 2-alkoxycarbonyl-1,3,4-triazol-5-yl in which the alkoxycarbonyl is 2-6C (e.g. methoxycarbonyl), each substituted in the 1-position:-

(a) by 1-6C alkyl (e.g. methyl), optionally substituted by 1-6C alkoxy (e.g. methoxy), 1-6C alkylthio (e.g. methylthio), phenyl, formyl, carbamoyl, 2-6C alkylcarbamoyl (e.g. methylcarbamoyl), 3-10C dialkylcarbamoyl

(e.g. dimethylcarbamoyl), 1-6C alkanoyl (e.g. acetyl), 2-6C alkoxycarbonyl (e.g. methoxycarbonyl) or thiazolidin-2-yl;

(b) by allyl, 2,3-dihydroxypropyl, 1,3-dihydroxyprop-2-yl, 2-formyl-2-hydroxyethyl, 3-formyloxy-2-hydroxypropyl, 2,3-bisformyloxypropyl or 1,3-bisformyloxyprop-2-yl;

(c) by 2-4C alkyl (e.g. ethyl) substituted by hydroxy, carbamoyloxy, 1-6C alkanoyl (e.g. acetyl) (which can itself be optionally substituted by amino, 1-6C alkylamino [e.g. methylamino] or 2-10C dialkylamino [e.g. dimethylamino]), 1-6C alkanesulphinyl (e.g. methanesulphinyl), 1-6C alkanesulphonyl (e.g. methanesulphonyl), amino, 1-6C alkylamino (e.g. methylamino), 2-10C dialkylamino (e.g. dimethylamino), sulphoamino, 1-6C alkanesulphonylamino, (e.g. methanesulphonylamino), sulphamoylamino, 1-6C alkanoylamino (e.g. acetylamino) (which can itself be optionally substituted by hydroxy, amino, 1-6C alkylamino [e.g. methylamino] or 2-10C dialkylamino [e.g. dimethylamino]), 2-6C alkoxycarbonylamino (e.g. methoxycarbonylamino), ureido, 2-6C alkylureido (e.g. methylureido), or 3-10C dialkylureido (e.g. dimethylureido);

(d) by a radical of the formula XVI, XVII or XVIII:-

[Formula XVI]

[Formula XVII]

[Formula XVIII]

in which alk is 1-4C alkylene (e.g. methylene), $Y^1$ and $Y^2$ are the same and are oxygen or sulphur and $R^{35}$ and $R^{36}$ are the same and are 1-6C alkyl (e.g. methyl), or $Y^1$ and $Y^2$ are the same or different and are oxygen or sulphur and $R^{35}$ and $R^{36}$ are joined to form 2-3C alkylene (e.g. ethylene), and $R^{37}$ is hydrogen or 1-3C alkyl (e.g. methyl);

(e) by 1-6C alkyl (e.g. methyl) substituted by 1-6C alkoxyimino (e.g. methoxyimino) or hydroxyimino;

5) 1,4-dialkyl-5,6-dioxo-1,4,5,6-tetrahydro-1,2,4-triazin-3-yl, 1-alkyl-5,6-dioxo-1,4,5,6-tetrahydro-1,2,4-triazin-3-yl or 2-alkyl-5,6-dioxo-1,2,5,6-tetrahydro-1,2,4-triazin-3-yl in each of which the alkyl is 1-6C (e.g. methyl);

6) 1,3,4-triazol-5-yl, 1,2,3-triazol-5-yl or 1-alkyl-1,2,4-triazol-5-yl in which the alkyl is 1-6C (e.g. methyl) which is optionally substituted in the 3-position by 2-6C alkoxycarbonyl (e.g. methoxycarbonyl);

7)a. 1,3,4-thiadiazol-5-yl optionally substituted by 1-6C alkyl (e.g. methyl), trifluoromethyl, 1-6C alkoxy (e.g. methoxy), 1-6C alkylthio (e.g. methylthio), 2-4C hydroxyalkylthio (e.g. 2-hydroxyethylthio), 1-6C alkanesulphonyl (e.g. methanesulphonyl), hydroxy, 1-6C hydroxyalkyl (e.g. hydroxymethyl), carboxy, 2-6C carboxyalkyl (e.g. carboxymethyl), amino, 1-6C alkylamino (e.g. methylamino), 2-10C dialkylamino (e.g. dimethylamino), 1-6C aminoalkyl (e.g. aminomethyl), 2-8C alkylaminoalkyl (e.g. methylaminomethyl), 3-12C dialkylaminoalkyl (e.g. dimethylaminomethyl), 1-6C alkanoylamino (e.g. acetylamino) or 2-8C alkanoylaminoalkyl (e.g. acetylaminomethyl), or

b. 1,2,4-thiadiazol-5-yl substituted by 1-6C alky (e.g. methyl) or 1-6C alkoxy (e.g. methoxy);

8)a. 1,3,4-oxadiazol-5-yl optionally substituted by 1-6C alkyl (e.g. methyl), trifluoromethyl, phenyl, 1-6C aminoalkyl (e.g. aminomethyl), 2-8C alkylaminoalkyl (e.g. methylaminomethyl), 3-10C dialkylaminoalkyl (e.g. dimethylaminomethyl) or 2-8C alkanoylaminoalkyl (e.g. acetylaminomethyl) or

b. oxazol-2-yl optionally substituted in the 4-position by 1-6C alkyl (e.g. methyl);

9) tetrazol-5-yl optionally substituted in the 1-position by:-

(a) 1-6C alkyl (e.g. methyl) itself optionally substituted by 1-6C alkoxy (e.g. methoxy), sulpho, carboxy, formyl or sulphamoyl;

(b) 2-4C alkyl (e.g. ethyl) substituted by hydroxy, amino, 1-6C alkylamino (e.g. methylamino), 2-8C dialkylamino (e.g. dimethylamino), 1-6C alkanoylamino (e.g. acetylamino), 2-6C carboxyalkylamino (e.g. carboxymethylamino), sulphamoylamino, sulphoamino, ureido, 2-6C alkylureido (e.g. methylureido) or 3-8C dialkylureido (e.g. dimethylureido);

(c) 1-5C alkyl (e.g. methyl) substituted by hydroxyimino or 1-6C alkoxyimino (e.g. methoxyimino);

(d) phenyl, 2,3-dihydroxypropyl, 1,3-dihydroxyprop-2-yl, 2-formyl-2-hydroxyethyl, 3-formyloxy-2-hydroxypropyl, 2,3-bisformyl-oxypropyl or 1,3-bisformyloxy-2-propyl;

or

(e) a radical of the formula XVI above in which $R^{37}$ is hydrogen, or a radical of the formula XVII above, in both of which $Y^1$, $Y^2$, $R^{35}$ and $R^{36}$ are as given above;

$R^2$ is carboxy, tetrazol-5-yl or a radical of the formula XIX, XX, XXI, XXII, XXIII, XXIV, XXV, XXVI, XXVII, XXVIII or XXIX:-

[Formula XIX]

[Formula XX]

[Formula XXI]

[Formula XXII]

[Formula XXIII]

[Formula XXIV]

[Formula XXV]

[Formula XXVI]

[Formula XXVII]

[Formula XXVIII]

[Formula XXIX]

in which $R^{38}$ is hydrogen or 1-6C alkyl, $R^{39}$ is 1-6C alkyl, $R^{40}$ is hydrogen, 1-6C alkyl, 7-11C arylalkyl or 2-6C alkoxycarbonyl, t is 0 or 1, $R^{41}$ is 1-6C alkyl, 6-10C aryl or 7-11C aralkyl, $R^{42}$ is hydrogen or one, two or three radicals selected from halogen, nitro, cyano, 1-6C alkyl, 1-6C alkoxy, 1-6C alkylthio, 1-6C alkylsulphinyl, 1-6C alkanesulphonyl, 2-6C alkoxycarbonyl, 2-6C alkoxythio-carbonyl, 2-6C alkanoylamino, 6-10C aryl, 6-10C aryloxy,

6-10C arylthio, 6-10C arylsulphinyl, 6-10C arylsulphonyl, 7-11C aryloxycarbonyl, 7-11C arylthiocarbonyl and 7-11C aryloxythiocarbonyl, $R^{43}$ is hydrogen or one of the values for $R^{41}$ given above and $R^{44}$ is hydrogen or one, two or three radicals selected from halogen, 1-6C alkyl and 1-6C alkoxy,

$X^1$ is sulphur, oxygen or $CH_2$;

$X^2$ is nitrogen or of the formula $\overset{\oplus}{N}-R^6$;

$R^3$ and $R^6$, which may be the same or different, are hydrogen, methyl, acetyl, hydroxy, methoxy, amino, acetylamino, methylamino, 2-aminoethyl, 2-hydroxyethyl, carboxymethyl, 2-carboxyethyl, 3-carboxypropyl, allyl, methoxymethyl, methoxycarbonylmethyl, t-butoxycarbonylmethyl, 3-(ethoxycarbonyl)propyl, 3-(t-butoxycarbonyl)propyl, furylmethyl, phenyl, benzyl,

in the latter two of which the phenyl ring is optionally suDstituted by fluorine, chlorine, bromine, methyl, methoxy, nitro, hyroxy, amino, carboxy or methoxycarbonyl,

propargyl, allenyl or 3-phenylallyl;

one of $R^4$ and $R^5$ is hydrogen, methyl or benzyl and the other is of the formula II:-

[Formula II]

in which n is 1 to 6 and $-R^7$ is of the formula III:-

[Formula III]

in which $R^8$ is 2-hydroxyethyl, 2-aminoethyl, 2-methylaminoethyl, 2-dimethylaminoethyl (and the N-oxide thereof), 2-cyanoethyl or 2-amino-2-carboxyethyl, or furyl, thienyl, pyrrolyl, oxazolyl, thiazolyl, imidazolyl, pyrazolyl, isoxazolyl, isothiazolyl, thiadiazolyl, oxadiazolyl, triazolyl, tetrazolyl, pyridyl, pyrimidyl, pyrazinyl or pyridazinyl, each optionally substituted by one or two substituents

selected from fluorine, chlorine, bromine, methyl, hydroxy, methoxy, mercapto, methylthio, carboxy, methoxycarbonyl, carbamoyl, methylcarbamoyl, dimethylcarbamoyl, nitro, amino, methylamino, dimethylamino, acetylamino, (acetyl)(methyl)amino, cyano, sulphamoyl, methylsulphamoyl, dimethylsulphamoyl, trifluoromethyl, methanesulphinyl and methanesulphonyl; or $-R^7$ is of the formula IV:-

[Formula IV]

in which $R^9$ is 3-aminopropyl, 3-(p-nitrobenzyloxycarbonylamino)propyl, 2-methoxyethyl, ethoxycarbonylmethyl, allyl, trifluoromethyl, carbamoylmethyl, methylcarbamoylmethyl, dimethylcarbamoylmethyl or 2-cyanoethyl, or phenyl, furyl, thienyl, pyrrolyl, oxazolyl, thiazolyl, imidazolyl, pyrazolyl, isoxazolyl, isothiazolyl, thiadiazolyl, oxadiazolyl, triazolyl, tetrazolyl, pyridyl, pyrimidyl, pyrazinyl or pyridazinyl each optionally substituted by one or two substituents selected from fluorine, chlorine, bromine, methyl, hydroxy, methoxy, mercapto, methylthio, carboxy, methoxycarbonyl, carbamoyl, methylcarbamoyl, dimethylcarbamoyl, nitro, amino, methylamino, dimethylamino, acetylamino, (acetyl)(methyl)amino, cyano, sulphamoyl, methylsulphamoyl, dimethylsulphamoyl, trifluoromethyl, methanesulphinyl and methanesulphonyl; or $-R^7$ is of the formula V:-

[Formula V]

in which $R^{10}$ is phenyl, furyl, thienyl, thiazolyl or thiadiazolyl, the latter two being optionally substituted by amino and $R^{11}$ is hydrogen, methyl, 1-carboxy-1-methylethyl or 1-ethoxycarbonyl-1-methylethyl;

or $-R^7$ is of the formula VI:-

[Formula VI]

in which $R^{12}$ is Gly, D-Ala, L-Ala, D-β-Ala, L-β-Ala, D-Val, L-Val, D-Leu, L-Leu, D-Ser, L-Ser, D-Thr, L-Thr, D-Arg, L-Arg, D-Lys, L-Lys, D-His, L-His, D-Asp, L-Asp, D-Glu, L-Glu, D-Asn, L-Asn, D-Gln, L-Gln, D-Lys, L-Lys, D-Met, L-Met, D-Phe, L-Phe, D-Tyr, L-Tyr, D-Trp, L-Trp, D-Pro, L-Pro or D-Ala-D-Ala, all the above residues optionally carrying a benzyloxycarbonyl, p-nitrobenzyloxycarbonyl, t-butoxycarbonyl or allyloxycarbonyl radical, the Glu and Asp residues optionally being in the form of the methyl or ethyl esters and the Arg residues optionally being in the form of the nitro derivative;

or $-R^7$ is of the formula VII:-

[Formula VII]

in which -A- is of the formula VIII:-

[Formula VIII]

in which p is 1 or 2, or A is 3,4-dioxocyclobuten-1,2-diyl or of the formula C=Z in which Z is NCN, $NCONH_2$, $NNO_2$, $CHNO_2$, $C(CN)_2$, $NCOR^{14}$, $NCO_2R^{14}$, $NSO_2R^{14}$ or $NR^{15}$ in which $R^{13}$ is methoxy, methylthio, amino, methylamino or dimethylamino, $R^{14}$ is methyl or phenyl and $R^{15}$ is methyl:

and where the compound of the formula I contains a free basic or acidic group, the pharmaceutically-acceptable acid- and base-addition salts thereof.

3. A cephalosporin derivative as claimed in claim 1 or 2 in which $X^1$ is sulphur, $R^0$ is hydrogen,

$R^1$ is hydrogen, chlorine, methyl, acetoxymethyl, 1-methyl-1H-tetrazol-5-ylthiomethyl, 1-carboxy-methyl-1H-tetrazol-5-ylthiomethyl, 1-(2-dimethylamino)-ethyl-1H-tetrazol-5-ylthiomethyl, 1-sulphomethyl-1H-tetrazol-5-ylthiomethyl, 1-isopropyl-1H-tetrazol-5-yl-thiomethyl, 1-(2,2,2-trifluoro)ethyl-1H-tetrazol-5-ylthiomethyl, 1-phenyl-1H-tetrazol-5-ylthiomethyl, 1-(2-methylthio)ethyl-1H-tetrazol-5-ylthiomethyl, 1,3,4-thiadiazol-2-ylthiomethyl, 5-methyl-1,3,4-thiadiazol-2-ylthiomethyl, 1,2,3-thiadiazol-5-ylthiomethyl, 1H-1,2,3-triazol-4-ylthiomethyl, 5-trifluoromethyl-1H-1,2,4-triazol-3-ylthiomethyl, 4,6-dimethylpyrimid-2-ylthiomethyl, 2-thiazolin-2-ylthiomethyl, benzoxazol-2-ylthiomethyl, benzthiazol-2-ylthiomethyl, 2-carboxyphenylthiomethyl, (6-carboxymethyl-7-hydroxypyrrolo-[1,2-b]pyridazin-2-yl)thiomethyl, methoxymethyl, hydroxymethyl, azidomethyl, aminomethyl, benzoyloxymethyl, acetylaminomethyl, carbamoyloxymethyl, 2-methylthio-1,3,4-thiadiazol-5-ylthiomethyl, 2-mercapto-1,3,4-thiadiazol-5-ylthiomethyl, 2-acetylamino-1,3,4-thiadiazol-5-ylthiomethyl, 5-methyl-1,2,4-thiadiazol-2-ylthiomethyl, 2-sulphomethyl-1,2,4-oxadiazol-5-ylthiomethyl, 4-methyl-5-(3-carboxypropyl)thiazol-2-ylthiomethyl, 2H-2-methyl-1,2,3-triazol-4-ylthiomethyl, 1H-1,2,4-triazol-2-ylthiomethyl, 4,5-dihydro-6-hydroxy-4-methyl-5-oxo-1,2,4-triazin-3-ylthiomethyl, 2,5-dihydro-6-hydroxy-2-methyl-5-oxo-1,2,4-triazin-3-ylthiomethyl, 1-oxidopyrid-2-ylthiomethyl, imidazo[4,5-b]pyrid-2-ylthiomethyl or imidazo[4,5-b]pyrimidin-2-ylthiomethyl, $R^2$ is carboxy, $X^2$ is nitrogen, $R^3$ is hydrogen, $R^4$ is hydrogen and $R^5$ is of the formula II in which $R^7$ is of the formula III or IV in which $R^8$ or $R^9$ is 1H-tetrazol-5-yl, 1H-1,2,3-triazol-4-yl or 1H-1,2,4-triazol-3-yl, each optionally substituted on nitrogen by methyl.

4. A cephalosporin derivative as claimed in claim 3 in which $R^1$ is 1-methyl-1H-tetrazol-5-ylthiomethyl.

5. A cephalosporin derivative selected from the group consisting of 7-(4-[3-(3-cyano-2-methylisothioureido)propyl]imidazol-2-yl)amino-3-(1-methyl-1H-tetrazol-5-yl)thiomethylceph-3-em-4-carboxylic acid, 7-(4-[3-(1-methyl-1H-tetrazol-5-ylthio)propyl]imidazol-2-yl)amino-3-(1-methyl-1H-tetrazol-5-yl)thiomethylceph-3-em-4-carboxylic acid, 7-(4-[3-(1-methyl-1H-tetrazol-5-ylsulphonylamino)propyl]imidazol-2-yl)amino-3-(1-methyl-1H-tetrazol-5-yl)thiomethylceph-3-em-4-carboxylic acid and the pharmaceutically-acceptable acid- and base-addition salts thereof.

6. A process for the manufacture of a cephalosporin derivative as claimed in claim 1, characterised by:-

(a) for those compounds in which $R^2$ is carboxy or a heterocyclic radical carrying an acidic proton and there is optionally carboxy in another part of the molecule, deprotection of the corresponding compound which carries a protecting group, or groups, in place of the acidic hydrogen atom, or atoms;

(b) reaction of a compound of the formula XXX:-

[Formula XXX]

with a compound of the formula XXXI:-

[Formula XXXI]

in which $R^{45}$ is a displaceable radical;

(c) for those compounds in which $R^1$ is a radical of the formula $CH_2Y$, reaction of a compound of the formula I in which $R^1$ is a radical of the formula $CH_2-R^{45}$ in which $R^{45}$ is a displaceable radical with a compound of the formula Y-H;

(d) for those compounds in which $R^8$ or $R^9$ is a radical which contains an NH group, deprotection of the corresponding compound which carries a protecting group in place of the hydrogen atom of the NH group;

(e) for those compounds in which $R^3$ is other than hydrogen, and $X^2$ is nitrogen, reaction of a compound of the formula XXX with a compound of the formula XXXII:-

[Formula XXXII]

in which $R^{46}$ has the value given above for $R^3$ other than hydrogen, $R^{47}$ is a displaceable radical and Z is an anion:

Whereafter, when the process of the invention manufactures the compound of the formula I in the form of the free acid or free base, or the zwitterion, and a salt is required, the compound of the formula I in the free acid or zwitterionic form is reacted with a base which affords a pharmaceutically-acceptable cation, or the compound of the formula I in the free base or zwitterionic form is reacted with an acid which affords a pharmaceutically-acceptable anion, and when the process of the invention manufactures the compound of the formula I in the form of an acid-addition salt and the zwitterionic form is required, the compound of the formula I in the form of the acid-addition salt is reacted with a low molecular weight epoxide such as epoxypropane.

7. A pharmaceutical composition which comprises a cephalosporin derivative as claimed in claim 1 in association with a pharmaceutically-acceptable diluent or carrier.

Claims for Austria

1. A process for the manufacture of a cephalosporin derivative of the formula I:-

[Formula I - given hereafter]

in which $R^0$ is hydrogen or methyl;
$R^1$ is any one of the C-3 substituents from antibacterially-active cephalosporins known in the art;
$R^2$ is any one of the C-4 substituents from antibacterially-active cephalosporins known in the art;
$X^1$ is sulphur, oxygen or $CH_2$;
$X^2$ is nitrogen or of the formula $\overset{\oplus}{N}-R^6$;
$R^3$ and $R^6$, which may be the same or different, are hydrogen 1-6C alkyl, 1-6C alkanoyl, hydroxy, 1-6C alkoxy, amino, 1-6C alkanoylamino, 1-6C alkylamino, 1-6C aminoalkyl, 1-6C hydroxyalkyl, 2-6C carboxyalkyl, 2-6C alkenyl, 3-6C alkoxyalkyl, 3-8C alkoxycarbonylalkyl, furylmethyl, phenyl, 7-11C phenylalkyl,

in the latter two of which the phenyl ring is optionally substituted by halogen, methyl, methoxy, nitro, hydroxy, amino, carboxy or methoxycarbonyl,

3-6C alkynyl, 3-6C alkadienyl or 9-16C arylalkenyl;
one of $R^4$ and $R^5$ is hydrogen, 1-6C alkyl or 7-11C aralkyl and the other is of the formula II:-

[Formula II]

in which n is 1 to 6 and $-R^7$ is of the formula III:-

[Formula III]

in which $R^8$ is 2-6C hydroxyalkyl, 2-6C aminoalkyl, 3-10C alkylaminoalkyl, 4-15C dialkylaminoalkyl (or the N-oxide thereof), 2-6C cyanoalkyl, 3-6C (amino)-(carboxy)alkyl or heteroaryl,
or $-R^7$ is of the formula IV:-

[Formula IV]

in which $R^9$ is 2-6C aminoalkyl, p-nitrobenzyloxycarbonyl(2-6C aminoalkyl), 2-8C alkoxyalkyl, 3-10C alkoxycarbonylalkyl, 2-6C alkenyl, 1-6C haloalkyl, 2-6C carbamoylalkyl, 3-10C alkylcarbamoylalkyl, 4-15C dialkylcarbamoylalkyl, 2-6C cyanoalkyl, 6-10C aryl or heteroaryl,
or $-R^7$ is of the formula V:-

[Formula V]

in which $R^{10}$ is 6-10C aryl, furyl, thienyl, thiazolyl or thiadiazolyl, the latter two being optionally substituted by amino, and $R^{11}$ is hydrogen, 1-6C alkyl, 2-8C carboxyalkyl or 3-10C alkoxycarbonylalkyl,
or $-R^7$ is of the formula VI:-

[Formula VI]

in which $R^{12}$ is an acyl radical derived from an aminoacid or from two or three aminoacids linked via normal peptide bonds, the aminoacids (apart from glycine) being in either the D or L configuration and being selected from glycine, alanine, -alanine, valine, leucine, isoleucine, serine, threonine, arginine, lysine, histidine, aspartic acid, glutamic acid, asparagine, glutamine, cysteine, methionine, phenylalanine, tyrosine, tryptophan and proline, the amino groups in such acyl radicals optionally carrying one or (where possible) more protecting groups selected from benzyloxycarbonyl, p-nitrobenzyloxycarbonyl, t-butoxycarbonyl and allyloxycarbonyl, the glutamic and aspartic acid residues optionally being in the form of the 1-6C alkyl esters and the arginine residue optionally being in the form of the nitro derivative,
or $-R^7$ is of the formula VII:-

[Formula VII]

in which -A- is of the formula VIII:-

[Formula VIII]

in which p is 1 or 2, or A is 3,4-dioxocyclobuten-1,2-diyl or of the formula C=Z in which Z is NCN, $NCONH_2$, $NNO_2$, $CHNO_2$ $C(CN)_2$, $NCOR^{14}$, $NCO_2R^{14}$, $SO_2R^{14}$ or $NR^{15}$ in which $R^{14}$ is 1-6C alkyl or 6-10C aryl and $R^{15}$ is 1-6C alkyl and $R^{13}$ is 1-6C alkoxy, 1-6C alkylthio, amino, 1-6C alkylamino or 2-10C dialkylamino;

wherein when $R^8$ or $R^9$ is heteroaryl it is a 5 or 6 membered heterocyclic aromatic ring containing 1,2,3 or 4 hetero atoms selected from oxygen, nitrogen and sulphur, and wherein when $R^8$ or $R^9$ is heteroaryl or $R^9$ is aryl the ring may be optionally substituted by one or two substituents selected from halogen, 1-6C alkyl, hydroxy, 1-6C alkoxy, mercapto, 1-6C alkylthio, carboxy, 2-6C alkoxycarbonyl, carbamoyl, 2-6C alkylcarbamoyl, 3-10C dialkylcarbamoyl, nitro, amino, 1-6C alkylamino, 2-8C dialkylamino, 1-6C alkanoylamino, 2-10C (alkanoyl)(alkyl)amino, cyano, sulphamoyl, 1-6C alkylsulphamoyl, 2-10C dialkylsulphamoyl, 1-6C haloalkyl, 1-6C alkanesulphinyl and 1-6C alkanesulphonyl;

provided that when $R^{10}$ is heteroaryl and m is 0 then n is 2 to 6;

and where the compound of the formula I contains a free basic or acidic group, the pharmaceutically-acceptable acid- and base-addition salts thereof, characterised by:-

32131 0082648

(a) for those compounds in which $R^2$ is carboxy or a heterocyclic radical carrying an acidic proton and there is optionally carboxy in another part of the molecule, deprotection of the corresponding compound which carries a protecting group, or groups, in place of the acidic hydrogen atom, or atoms;

(b) reaction of a compound of the formula XXX:-

[Formula XXX]

with a compound of the formula XXXI:-

[Formula XXXI]

in which $R^{45}$ is a displaceable radical;

(c) for those compounds in which $R^1$ is a radical of the formula $CH_2Y$, reaction of a compound of the formula I in which $R^1$ is a radical of the formula $CH_2-R^{45}$ in which $R^{45}$ is a displaceable radical with a compound of the formula Y-H;

(d) for those compounds in which $R^8$ or $R^9$ is a radical which contains an NH group, deprotection of the corresponding compound which carries a protecting group in place of the hydrogen atom of the NH group;

(e) for those compounds in which $R^3$ is other than hydrogen, and $X^2$ is nitrogen, reaction of a compound of the formula XXX with a compound of the formula XXXII:-

[Formula XXXII]

in which $R^{46}$ has the value given above for $R^3$ other than hydrogen, $R^{47}$ is a displaceable radical and Z is an anion:

Whereafter, when the process of the invention manufactures the compound of the formula I in the form of the free acid or free base, or the zwitterion, and a salt is required, the compound of the formula I in the free acid or zwitterionic form is reacted with a base which affords a pharmaceutically-acceptable cation, or the compound of the formula I in the free base or zwitterionic form is reacted with an acid which affords a pharmaceutically-acceptable anion, and when the process of the invention manufactures the compound of the formula I in the form of an acid-addition salt and the zwitterionic form is required, the compound of the formula I in the form of the acid-addition salt is reacted with a low molecular weight epoxide such as epoxypropane.

IJSB/YB : PH.32131/OE : 2 Dec 82
SA/09

IVOR JAMES STEWART BROWN

AUTHORISED REPRESENTATIVE
General Authorisation No. 97

0082648

Imperial Chemical Industries PLC — Formula Drawings
"Cephalosporin Derivatives" — 3 Sheets
Sheet 1

1/3

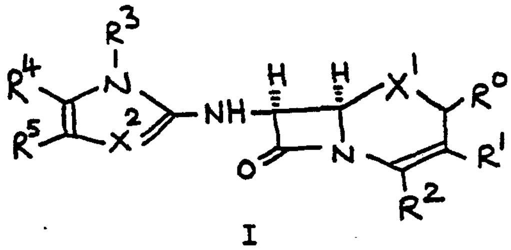

I

$R^7-(CH_2)_n-$

II

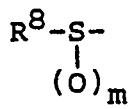

III

$R^9SO_2NH-$

IV

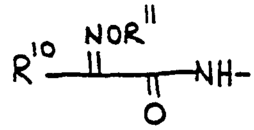

V

$R^{12}-NH-$

VI

$R^{13}-A-NH-$

VII

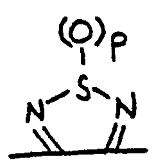

VIII

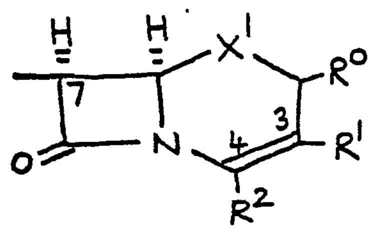

IX

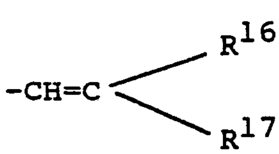

X

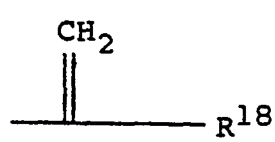

XI

Imperial Chemical Industries PLC
"Cephalosporin Derivatives"

Formula Drawings
3 Sheets
Sheet 2

2/3

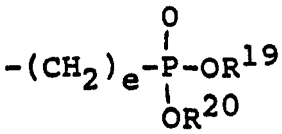

XII

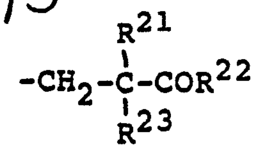

XIII

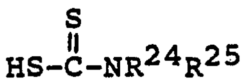

XIV

$-CH=CH-S-R^{34}$

XV

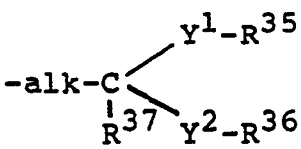

XVI

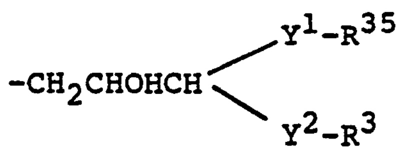

XVII

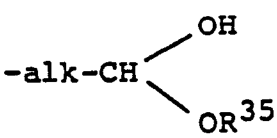

XVIII

$-COOCHR^{38}OCOR^{39}$

XIX

$-COOCHR^{38}SCOR^{39}$

XX

$-COOCHR^{38}OR^{39}$

XXI

$-COOCOOR^{39}$

XXII

$-COOCHR^{38}OCOR^{39}$

XXIII

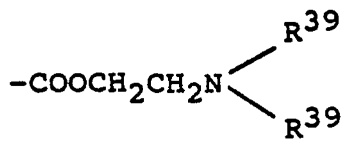

XXIV

$-COOCHR^{38}OCH_2CH_2OCH_3$

XXV

$-COOCH_2OCO(CH_2)_t-CHR^{40}-NH_2$

XXVI

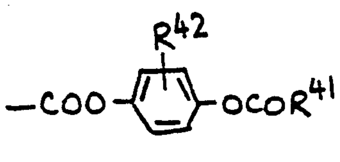

XXVII

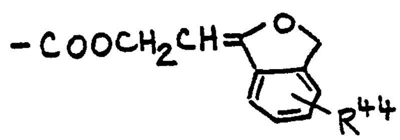

XXVIII

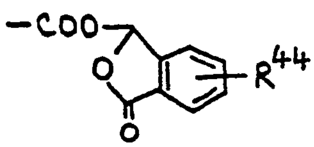

XXIX

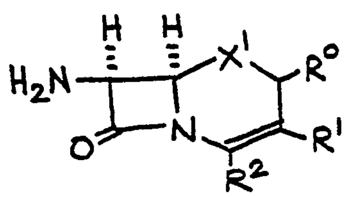

XXX

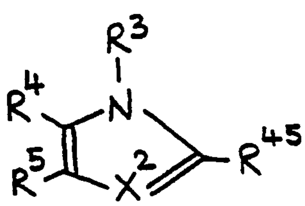

XXXI

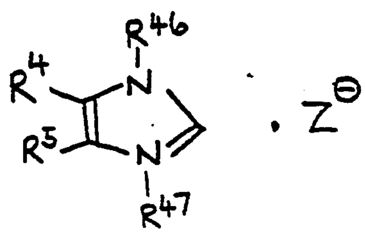

XXXII